(19) Europäisches Patentamt
European Patent Office
Office européen des brevets

(11) **EP 3 228 320 B1**

(12) **EUROPÄISCHE PATENTSCHRIFT**

(45) Veröffentlichungstag und Bekanntmachung des Hinweises auf die Patenterteilung:
**18.12.2019 Patentblatt 2019/51**

(51) Int Cl.:
*A61K 38/28* *(2006.01)* *A61K 38/26* *(2006.01)*
*A61P 5/50* *(2006.01)*

(21) Anmeldenummer: **17169192.6**

(22) Anmeldetag: **09.10.2009**

(54) **KOMBINATION VON EINEM INSULIN UND EINEM GLP-1-AGONISTEN**

COMBINATION OF AN INSULIN AND A GLP-1 AGONIST

COMBINAISON D'UNE INSULINE ET D'UN ANTAGONISTE GLP 1

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(30) Priorität: **17.10.2008 DE 102008051834**
**24.10.2008 DE 102008053048**
**20.08.2009 DE 102009038210**

(43) Veröffentlichungstag der Anmeldung:
**11.10.2017 Patentblatt 2017/41**

(60) Teilanmeldung:
**19203765.3**

(62) Dokumentnummer(n) der früheren Anmeldung(en) nach Art. 76 EPÜ:
**09783905.4 / 2 349 324**

(73) Patentinhaber: **Sanofi-Aventis Deutschland GmbH**
**65929 Frankfurt am Main (DE)**

(72) Erfinder:
• **WERNER, Ulrich**
  **65926 Frankfurt am Main (DE)**
• **ROTTHÄUSER, Bärbel**
  **65926 Frankfurt am Main (DE)**
• **SMITH, Christopher, James**
  **65926 Frankfurt am Main (DE)**

(74) Vertreter: **Weickmann & Weickmann PartmbB**
**Postfach 860 820**
**81635 München (DE)**

(56) Entgegenhaltungen:
**WO-A2-03/020201**

• **DATABASE BIOSIS [Online] BIOSCIENCES INFORMATION SERVICE, PHILADELPHIA, PA, US; März 2008 (2008-03), TEWS D ET AL: "Enhanced protection against cytokine- and fatty acid-induced apoptosis in pancreatic beta cells by combined treatment with glucagon-like peptide-1 receptor agonists and insulin analogues", XP002577467, Database accession no. PREV200800343949 & HORMONE AND METABOLIC RESEARCH, Bd. 40, Nr. 3, März 2008 (2008-03), Seiten 172-180, ISSN: 0018-5043**

EP 3 228 320 B1

**Beschreibung**

Die Erfindung betrifft eine pharmazeutische Kombination umfassend (a) Gly(A21)-Arg(B31)-Arg(B32)-Humaninsulin oder/und ein pharmakologisch tolerierbares Salz davon in einer Konzentration von 40 bis 500 U/ml, und (b) desPro$^{36}$Exendin-4(1-39)-Lys$_6$-NH$_2$ oder/und ein pharmakologisch tolerierbares Salz davon in einer Konzentration von 10 bis 300 μg/ml.

In einem weiteren Aspekt betrifft die Erfindung eine pharmazeutische Zusammensetzung umfassend (a) Gly(A21)-Arg(B31)-Arg(B32)-Humaninsulin oder/und ein pharmakologisch tolerierbares Salz davon in einer Konzentration von 40 bis 500 U/ml, und (b) desPro$^{36}$Exendin-4(1-39)-Lys$_6$-NH$_2$ oder/und ein pharmakologisch tolerierbares Salz davon in einer Konzentration von 10 bis 300 μg/ml.

Weltweit leiden etwa 250 Mio. Menschen an Diabetes mellitus. Für die Typ 1 Diabetiker darunter ist die Substitution der fehlenden endokrinen Insulinsekretion die einzige derzeit mögliche Therapie. Die Betroffenen sind lebenslang, in der Regel mehrmals täglich, auf Insulininjektionen angewiesen. Im Gegensatz zum Typ 1-Diabetes besteht beim Typ 2-Diabetes nicht grundsätzlich ein Mangel an Insulin, jedoch wird in einer Vielzahl von Fällen, vor allem im fortgeschrittenen Stadium, die Behandlung mit Insulin, gegebenenfalls in Kombination mit einem oralen Antidiabetikum, als günstigste Therapieform angesehen.

Beim Gesunden ist die Insulinfreisetzung durch das Pankreas strikt an die Konzentration der Blutglukose gekoppelt. Erhöhte Blutglukosespiegel, wie sie nach Mahlzeiten auftreten, werden durch eine entsprechende Steigerung der Insulinsekretion rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glukose zu gewährleisten und die hepatische Glukoseproduktion in der Nacht niedrig zu halten. Der Ersatz der körpereigenen Insulinsekretion durch exogene, meist subkutane Applikation von Insulin erreicht in der Regel die oben beschriebene Qualität der physiologischen Regulation der Blutglukose nicht annähernd. Häufig kommt es zu Entgleisungen der Blutglukose nach oben oder unten, die in ihren schwersten Formen lebensbedrohlich sein können. Daneben stellen jedoch auch über Jahre erhöhte Blutglukosespiegel ohne anfängliche Symptome ein erhebliches Gesundheitsrisiko dar. Die großangelegte DCCT-Studie in den USA (The Diabetes Control and Complications Trial Research Group (1993) N. Engl. J. Med. 329, 977-986) wies eindeutig nach, daß chronisch erhöhte Blutglukosespiegel wesentlich für die Entwicklung diabetischer Spätschäden verantwortlich sind. Diabetische Spätschäden sind mikro- und makrovaskuläre Schädigungen, die sich u.U. als Retino-, Nephro-, oder Neuropathie manifestieren und zu Erblindung, Nierenversagen sowie dem Verlust von Extremitäten führen und darüber hinaus mit einem erhöhten Risiko für Herz/Kreislauferkrankungen einhergehen. Daraus ist abzuleiten, dass eine verbesserte Therapie des Diabetes in erster Linie darauf abzielen muss, die Blutglukose möglichst eng im physiologischen Bereich zu halten. Nach dem Konzept der intensivierten Insulintherapie soll dies durch mehrmals tägliche Injektionen von schnell und langsam wirkenden Insulinzubereitungen erreicht werden. Rasch wirkende Formulierungen werden zu den Mahlzeiten gegeben, um den postprandialen Anstieg der Blutglukose auszugleichen. Langsam wirkende Basalinsuline sollen die Grundversorgung mit Insulin insbesondere während der Nacht sicherstellen, ohne zu einer Hypoglykämie zu führen.

Insulin ist ein Polypeptid aus 51 Aminosäuren, die sich auf 2 Aminosäureketten verteilen: die A Kette mit 21 Aminosäuren und die B-Kette mit 30 Aminosäuren. Die Ketten sind durch 2 Disulfidbrücken miteinander verbunden. Insulinzubereitungen werden seit vielen Jahren zur Diabetestherapie eingesetzt. Dabei werden nicht nur natürlich vorkommende Insuline verwendet, sondern neuerdings auch Insulinderivate und -analoga.

Insulinanaloga sind Analoga von natürlich vorkommenden Insulinen, nämlich Humaninsulin oder tierischen Insulinen, welche sich durch Substitution wenigstens eines natürlich auftretenden Aminosäurerestes mit anderen Aminosäuren und/oder Addition/Entfernen wenigstens eines Aminosäurerestes von dem entsprechenden, ansonsten gleichen natürlich vorkommenden Insulin unterscheiden. Es kann sich dabei auch um Aminosäuren handeln, die nicht natürlich vorkommen.

Insulinderivate sind Derivate von natürlich vorkommendem Insulin oder einem Insulinanalogon, welche durch chemische Modifizierung erhalten werden. Die chemische Modifikation kann z.B. in der Addition einer oder mehrerer bestimmter chemischer Gruppen an eine oder mehrere Aminosäuren bestehen. In der Regel haben Insulinderivate und Insulinanaloga gegenüber humanem Insulin eine etwas veränderte Wirkung.

Insulinanaloga mit beschleunigtem Wirkungseintritt werden in EP 0 214 826, EP 0 375 437 und EP 0 678 522 beschrieben. EP 0 214 826 bezieht sich u.a. auf Substitutionen von B27 und B28. EP 0 678 522 beschreibt Insulinanaloga, die in der Position B29 verschiedene Aminosäuren, vorzugsweise Prolin, aufweisen, jedoch nicht Glutaminsäure. EP 0 375 437 umfaßt Insulinanaloga mit Lysin oder Arginin in B28, die optional zusätzlich in B3 und/oder A21 modifiziert sein können. Eine beschleunigte Wirkung weisen auch die in der EP-A-0 885 961 beschriebenen Insulinanaloga auf.

WO 2005/046716 offenbart eine pharmazeutische Zusammensetzung umfassend ein insulinotropes Peptid (Liraglutid) und ein mahlzeitbezogenes (schnellwirkendes) Insulinpeptid (Insulin Aspart).

In der EP 0 419 504 werden Insulinanaloga offenbart, die gegen chemische Modifikationen geschützt sind, indem Asparagin in B3 und wenigstens eine weitere Aminosäure in den Positionen A5, A15, A18 oder A21 verändert sind.

In der WO 92/00321 werden Insulinanaloga beschrieben, bei denen wenigstens eine Aminosäure der Positionen B1-B6 durch Lysin oder Arginin ersetzt ist. Derartige Insuline weisen gemäß WO 92/00321 eine verlängerte Wirkung auf. Eine verzögerte Wirkung weisen auch die in der EP-A 0 368 187 und die in den deutschen Patentanmeldungen Nr. 10 2008 003 568.8 und 10 2008 003 566.1 und der internationalen Anmeldung WO 2009/087081 beschriebenen Insulinanaloga auf.

WO 2006/051103 offenbart eine lagerstabile pharmazeutische Zusammensetzung umfassend ein insulinotropes Peptid, ein Basalinsulin, ein pharmazeutisch akzeptables Konservierungsmittel, eine zwitterionische oberflächenaktive Substanz und ein Poloxamer oder Polysorbat 20 bei einem pH von etwa 7.0 bis etwa 8.5.

WO 2003/020201 offenbart eine pharmazeutische Zusammensetzung umfassend Exendin-4 und Insulin Glargin.

Die auf dem Markt befindlichen Insulinzubereitungen von natürlich vorkommenden Insulinen zur Insulinsubstitution unterscheiden sich in der Herkunft des Insulins (z.B. Rind, Schwein, Humaninsulin), sowie der Zusammensetzung, womit das Wirkprofil (Wirkeintritt und Wirkdauer) beeinflusst werden kann. Durch Kombination verschiedener Insulinpräparate lassen sich unterschiedlichste Wirkprofile erzielen und möglichst physiologische Blutzuckerwerte einstellen. Die rekombinante DNA Technologie ermöglicht heutzutage die Herstellung von solchen modifizierten Insulinen. Hierzu zählt Insulin Glargin (Gly(A21)-Arg(B31)-Arg(B32)-Humaninsulin) mit einer verlängerten Wirkdauer. Insulin Glargin wird als saure, klare Lösung injiziert und fällt aufgrund seiner Lösungseigenschaften im physiologischen pH-Bereich des Subkutangewebes als stabiles Hexamerassoziat aus. Insulin Glargin wird einmal täglich injiziert und zeichnet sich gegenüber anderen langwirksamen Insulinen durch sein flaches Serumprofil und die damit verbundene Reduktion der Gefahr nächtlicher Hypoglykämien aus (Schubert-Zsilavecz et al., 2:125-130(2001)).

Die spezifische Zubereitung des Insulin Glargins, die zur verlängerten Wirkdauer führt, ist durch einen klare Lösung mit saurem pH-Wert gekennzeichnet.

Glucagon-like peptide 1 (GLP-1) ist ein endokrines Hormon, welches die Insulinantwort nach oraler Aufnahme von Glukose oder Fett erhöht. GLP-1 reguliert generell die Glukagonkonzentrationen, verlangsamt die Magenentleerung, stimuliert die (Pro-)Insulin-Biosynthese, erhöht die Empfindlichkeit gegenüber Insulin und stimuliert die insulinunabhängige Glykogen-Biosynthese (Holst (1999), Curr. Med. Chem 6:1005, Nauck et al. (1997) Exp Clin Endocrinol Diabetes 105: 187, Lopez-Delgado et al. (1998) Endocrinology 139:2811).

Humanes GLP-1 weist 37 Aminosäurereste auf (Heinrich et al., Endocrinol. 115:2176 (1984), Uttenthal et al., J Clin Endocrinol Metabol (1985) 61:472). Aktive Fragmente von GLP-1 schließen GLP-1 (7-36) amid und GLP-1(7-37) ein.

Exendine sind eine andere Gruppe von Peptiden, welche die Blutglukosekonzentrationen senken können. Exendine weisen eine gewisse Ähnlichkeit der Sequenz zu GLP-1(7-36) auf (53%, Goke et al. J. Biol Chem 268, 19650-55). Exendin-3 und Exendin-4 stimulieren einen Anstieg der zellulären cAMP-Produktion in Azinuszellen des Meerschweinchenpankreas durch Interaktion mit Exendinrezeptoren (Raufman, 1996, Reg. Peptides 61:1-18). Exendin-3 bewirkt im Gegensatz zu Exendin-4 einen Anstieg der Amylasefreisetzung in Azinuszellen des Pankreas.

Exendin-3, Exendin-4 und Exendinagonisten wurden für die Behandlung von Diabetes mellitus und die Prävention von Hyperglykämie vorgeschlagen, wobei sie die Magenmotilität und -entleerung vermindern (US 5,424,286 und WO98/05351).

Exendin-Analoga können gekennzeichnet sein durch Aminosäuresubstitutionen und/oder C-terminalen Trunkierung der nativen Exendin-4-Sequenz. Derartige Exendin-Analoga sind beschrieben in WO 99/07404, WO 99/25727, WO 99/25728.

Kombinationen von Insulin und GLP-1 sind aus WO 2004/005342 zur Behandlung von Diabetes bekannt.

Tews et al. (Horm Metab Res 40:172-180, 2008) offenbart eine erhöhte Protektion gegen Cytokin- oder Fettsäure-induzierte Apoptose in pankreatischen β-Zellen durch eine kombinierte Behandlung mit GLP-1-Rezeptoragonisten und Insulinanaloga in einem zellulären in-vitro-Testsystem.

In der klinischen Praxis wird die zu verabreichende Insulinmenge auf den individuellen Bedarf der einzelnen Diabetespatienten eingestellt. Individuelle Patienten benötigen in der Regel jeweils unterschiedliche Mengen an Insulin oder/und GLP-1-Agonist. Üblicherweise wird die vorbestimmte Dosis verabreicht, indem eine bestimmte Menge einer Zusammensetzung mit definierter Konzentration verabreicht wird. Hieraus ergibt sich, dass eine Zusammensetzung, welche Insulin und GLP-1 gleichzeitig enthält, die Verabreichung von nur einem bestimmten Verhältnis von Insulin und GLP-1 ermöglicht. Dies bedeutet, dass nur eine der beiden Mengen von Insulin und GLP-1 optimal an den Bedarf des Patienten angepasst werden kann. Da in der Praxis die korrekte Einstellung der verabreichten Menge von Insulin wesentlich ist, ist davon auszugehen, das bei Verabreichung von einem bestimmten Verhältnis von Insulin zu GLP-1 der GLP-1-Agonist entweder unter- oder überdosiert ist und allenfalls zufällig korrekt ist.

Es sind verschiedene Systeme zur Injektion einer Wirkstoffkombination bekannt. Die Wirkstoffe können in einer Zusammensetzung formuliert werden und in einer Vorrichtung, z.B. in einer Fertigspritze, bereitgestellt werden. Ein solches System ermöglicht zwar die Dosierung der Kombination, jedoch nur in einem fixen Verhältnis der Wirkstoffe, wie es in der Zusammensetzung enthalten ist. Wie hierin dargestellt, ist dies für die Kombination von einem Insulin mit einem GLP-1-Agonisten nachteilig, da unterschiedliche Mengen des Insulins und des GLP-1-Agonisten je nach therapeutischem Bedarf verabreicht werden müssen.

Ebenso können zwei Wirkstoffe in zwei getrennten Formulierungen verabreicht werden, welche jeweils einen der beiden Wirkstoffe enthalten und welche unabhängig voneinander mit jeweils einer Vorrichtung (z.B. Fertigspritzen) injiziert werden. Bei einer Injektionstherapie wie z.B. der Injektion von Insulin ist die Compliance des Patienten eine wesentliche Voraussetzung für den Erfolg der Therapie. Generell sind bei einer Injektionstherapie der Schmerz, eine Nadelphobie, die Mitnahmemöglichkeit für die Injektionsvorrichtung ein Problem, was zu einer verminderten Compliance führen kann. Soll der Patient zwei getrennte Vorrichtungen zur Injektion benutzen, so vergrößern sich diese Probleme.

Eine einzige Vorrichtung zur Verabreichung von Insulin und einem GLP-1-Agonisten ist gegenüber dem Einsatz von zwei getrennten Vorrichtungen zur Verabreichung von Insulin und einem GLP-1-Agonisten für den Patienten/Verwender vorteilhaft. Außerdem kann die Verwendung von nur einer Vorrichtung anstatt von zwei Vorrichtungen die Anzahl der Schritte reduzieren, welche der Patient/Verwender durchführen muss, was die Häufigkeit von Fehlern bei der Anwendung vermindert. Damit wird das Risiko für unerwünschte Nebenwirkungen reduziert.

US 4,689,042, US 5,478,323, US 5,253,785 und WO 01/02039 beschreiben Vorrichtungen zur gleichzeitigen Verabreichung von zwei injizierbaren Produkten an einen Patienten. Diese Vorrichtungen enthalten zwei Behälter, welche jeweils eine Zusammensetzung enthalten. In diesen Vorrichtungen erfolgt die Injektion beider Zusammensetzungen über eine Nadel. Damit können zwar die Nachteile überwunden werden, welche mit der Verwendung von zwei separaten Vorrichtungen entstehen. Durch die Mischung verdünnen sich die Konzentrationen der beiden Wirkstoffe. Dies kann sich nachteilig auf die Pharmakokinetik auswirken.

Die Pharmakokinetik von Insulin, insbesondere von Insulin Glargin, wird durch die Verdünnung des Insulins in der verabreichten Zusammensetzung beeinflusst. Um eine zuverlässige Wirkung einer bestimmten Dosis von Insulin sicherzustellen, sollte daher die Konzentration von Insulin möglichst konstant gehalten werden. Die Dosierung sollte im wesentlichen über das Volumen der verabreichten Insulinzusammensetzung erfolgen. Dies gilt auch für die Verabreichung einer Kombination von Insulin und einem GLP-1-Agonisten. Bei Verabreichung einer Kombination von Insulin und einem GLP-1-Agonisten ist diese Vorgabe nur zu erfüllen, wenn beide Substanzen in einer Zusammensetzung in einem fixen Verhältnis zueinander dosiert werden. Werden beide Substanzen in getrennten Zusammensetzungen bereitgestellt und in einer geeigneten Vorrichtung (z.B. aus WO 01/02039) zur Injektion gemischt, so läßt sich eine konstante Insulinkonzentration nur verwirklichen, wenn die Insulinzusammensetzung durch die Zusammensetzung des GLP-1-Agonisten nicht wesentlich verdünnt wird. Damit ist eine unabhängige Dosierung des Insulins und des GLP-1-Agonisten nur eingeschränkt möglich.

Eine denkbare Lösung wäre, den GLP-1-Agonisten in einer so hohen Konzentration bereitzustellen, dass die Zudosierung des GLP-1-Agonisten keine nennenswerte Verdünnung der Insulinzusammensetzung bewirkt (z.B. nicht mehr als 10%). Polypeptide wie Insuline (z.B. Insulin Glagin, Lantus®) oder GLP-1-Agonisten können nicht beliebig konzentriert werden. Zum einen ist die Löslichkeit von Proteinen begrenzt, und hohe Konzentrationen von Proteinen können das Fließverhalten der Lösung verändern. Das für die Anwendung von Lösungen mit hoher Wirkstoffkonzentration wichtigste Problem ist die Dosierungsgenauigkeit. Bei hohen Konzentrationen müssten kleine Volumina verabreicht oder zu einer anderen Lösung hinzudosiert werden. Vorrichtungen zur genauen Dosierung kleiner oder kleinster Volumina sind zwar bekannt. Diese sind jedoch teuer und aufgrund ihrer Handhabung nur für den Gebrauch durch geschultes Personal vorgesehen, z.B. im Labor. Da sich die Patienten die Insuline oder/und GLP-1-Agonisten in der Regel selbst injizieren, scheidet der Einsatz derartiger Vorrichtungen zur Verabreichung von Insulinen oder/und GLP-1-Agonisten aus. Die zum Beispiel in US 4,689,042, US 5,478,323, US 5,253,785 und WO 01/02039 beschriebenen Vorrichtungen, mit welchen sich der Patient Wirkstofflösungen selbst injizieren kann, sind zur Dosierung kleiner und kleinster Volumina nicht geeignet.

Für die Injektion einer Kombination eines Insulins und eines GLP-1-Agonisten stellen sich die folgenden Probleme:

- das Verhältnis der Wirkstoffe muss variabel sein,
- die Pharmakokinetik von mindestens einem der Wirkstoffe (des Insulins) wird durch die Konzentration/Verdünnung beeinflusst,
- die Pharmakokinetik von mindestens einem anderen Wirkstoff (des GLP-1-Agonisten) wird durch die Konzentration/Verdünnung nicht oder nicht wesentlich beeinflusst.

Der vorliegenden Erfindung lag somit die Aufgabe zugrunde, ein Arzneimittel bereitzustellen, welches die oben beschriebenen Nachteile des Standes der Technik zumindest teilweise überwindet. Ferner soll erreicht werden, dass möglichst nur eine Verabreichung pro Tag erfolgen soll.

Es wurde überraschenderweise gefunden, dass die Kombination von einem Insulin (Gly(A21)-Arg(B31)-Arg(B32)-Humaninsulin) mit einem GLP-1-Agonisten (desPro$^{36}$Exendin-4(1-39)-Lys$_6$-NH$_2$) synergistische Wirkungen bei der Regulation der Blutglukose in der postprandialen und postabsorptiven Phase aufweist verglichen mit der Anwendung von Insulin oder dem GLP-1-Agonisten allein:

- Höhere Wirksamkeit aufgrund der Kombination der komplementären Wirkungen auf die nüchternen und postprandialen Glukosewerte, welche sich ergänzen (Beispiele 2 und 3). Die Kombination zeigt eine Senkung der postprandialen Glukosekonzentration (=verbesserte Glukosetoleranz) wie ein GLP-1-Agonist allein und zusätzlich die postabsorptiven Glukosesenkung wie ein Insulin (Beispiel 9).
- Verminderung des Risikos einer Hypoglykämie (Beispiele 2-4).
- Bessere Anpassung der Blutglukosekonzentration an normoglykämische Werte (Beispiel 8).
- Verbesserte Glukosetoleranz und Absenkung der postabsorptiven Glukosekonzentrationen (Beispiel 9).
- Die synergistischen Wirkungen der Kombination auf die Glukosekonzentration werden in einem Konzentrationsbereich des GLP-1-Agonisten von einer Größenordnung (Faktor 10) beobachtet. (Beispiel 6 verglichen mit den Beispielen 4 und 2). Erst bei kleineren GLP-1-Dosen bzw. größeren Verhältnissen von Insulin zu GLP-1 überwiegen die Wirkungen des Insulins.
- Erhalt der Funktion der β-Zellen (Beispiel 10).
- Gewichtsverlust/Verminderung der Gewichtszunahme.
- Alle Beispiele zeigen, dass GLP-1-Agonisten und Insuline keine negativen Wechselwirkungen zeigen.
- Durch die Wirkungen auf die nüchternen, postprandialen und postabsorptiven Blutglukosekonzentrationen wird es möglich, die Zahl der Verabreichungen der Kombination auf einmal täglich zu reduzieren.

Hierin ist ein Arzneimittel beschrieben, umfassend wenigstens ein Insulin und wenigstens einen GLP-1-Agonisten, wobei das Arzneimittel so formuliert oder/und konfektioniert ist, dass es das Insulin und den GLP-1-Agonisten in einer jeweils vorbestimmten Menge enthält und in einer jeweils an den Bedarf eines Patienten angepassten Dosis verabreicht werden kann.

Ein erfindungsgemäßer Gegenstand ist eine pharmazeutische Kombination umfassend (a) Gly(A21)-Arg(B31)-Arg(B32)-Humaninsulin oder/und ein pharmakologisch tolerierbares Salz davon in einer Konzentration von 40 bis 500 U/ml, und (b) desPro$^{36}$Exendin-4(1-39)-Lys$_6$-NH$_2$ oder/und ein pharmakologisch tolerierbares Salz davon in einer Konzentration von 10 bis 300 $\mu$g/ml.

Ein weiterer erfindungsgemäßer Gegenstand ist eine pharmazeutische Zusammensetzung umfassend (a) Gly(A21)-Arg(B31)-Arg(B32)-Humaninsulin oder/und ein pharmakologisch tolerierbares Salz davon in einer Konzentration von 40 bis 500 U/ml, und (b) desPro$^{36}$Exendin-4(1-39)-Lys$_6$-NH$_2$ oder/und ein pharmakologisch tolerierbares Salz davon in einer Konzentration von 10 bis 300 $\mu$g/ml.

Die erfindungsgemäße Kombination oder Zusammensetzung wird insbesondere zur Behandlung von Patienten mit Diabetes mellitus verwendet, insbesondere von Patienten mit Diabetes Typ 1 oder Typ 2.

Durch die erfindungsgemäße Kombination oder Zusammensetzung kann die Blutglukosekonzentration bei Patienten mit Diabetes, insbesondere Diabetes Typ 1 oder Typ 2, besser an normoglykämische Werte angepasst werden.

Bevorzugt wird die erfindungsgemäße Kombination oder Zusammensetzung zur Einstellung der nüchternen, der postprandialen oder/und der postabsorptiven Blutglukosekonzentration von Patienten mit Diabetes verwendet, insbesondere von Patienten mit Diabetes Typ 1 oder Typ 2. Stärker bevorzugt wird die erfindungsgemäße Kombination oder Zusammensetzung zur Einstellung der postprandialen oder/und der postabsorptiven Blutglukosekonzentration von Patienten mit Diabetes verwendet, insbesondere von Patienten mit Diabetes Typ 1 oder Typ 2 verwendet. Einstellung bedeutet in diesem Zusammenhang, dass normoglykämische Blutglukosekonzentrationen im wesentlichen erreicht werden oder zumindest eine Annäherung daran erzielt wird. Unter normoglykämischen Werten werden insbesondere Blutglukosekonzentrationen im Normbereich (Schwankungsbreite 60 - 140 mg/dl entsprechend 3,3 bis 7,8 mmol/l) verstanden. Dieser Schwankungsbereich umfasst Blutglukosekonzentrationen unter Nüchternbedingungen, postprandialen und postabsorptiven Bedingungen.

Postprandial und postabsorptiv sind dem Fachmann auf dem Gebiet der Diabetologie geläufige Begriffe. Unter postprandial wird hierin insbesondere die Phase nach einer Mahlzeit oder/und nach einer Glukosebelastung im Experiment bezeichnet. Diese Phase ist insbesondere beim Gesunden durch einen Anstieg und den Wiederabfall der Glukosekonzentration im Blut gekennzeichnet. Als postabsorptiv oder postabsorptive Phase wird hierin insbesondere die sich an die postprandiale Phase anschließende Phase bezeichnet. Die postprandiale Phase endet typischerweise bis zu 4 h nach der Mahlzeit oder/und Glukosebelastung. Die postabsorptive Phase dauert typischerweise bis zu 8 bis 16 h.

Ebenso wird die erfindungsgemäße Kombination oder Zusammensetzung bevorzugt zur Verbesserung der Glukosetoleranz bei Behandlung eines Patienten mit Diabetes, insbesondere mit einem Diabetes Typ 1 oder Typ 2, verwendet. Unter Verbesserung der Glukosetoleranz wird verstanden, dass durch die erfindungsgemäße Kombination oder Zusammensetzung die postprandiale Blutglukosekonzentration gesenkt wird. Ebenso wird unter Verbesserung der Glukosetoleranz verstanden, dass durch die erfindungsgemäße Kombination oder Zusammensetzung die postabsorptive Blutglukosekonzentration gesenkt wird. Senkung bedeutet insbesondere, dass die Blutglukosekonzentration normoglykämische Werte im wesentlichen erreicht oder zumindest daran angenähert wird.

Die erfindungsgemäße Kombination oder Zusammensetzung kann das Risiko einer Hypoglykämie senken, welche z. B. in der postabsorptiven Phase auftreten kann. Die erfindungsgemäße Kombination oder Zusammensetzung wird bevorzugt zur Prävention einer Hypoglykämie bei Behandlung eines Patienten mit Diabetes, insbesondere mit einem Diabetes Typ 1 oder Typ 2, verwendet, wobei die Hypoglykämie insbesondere in der postabsorptiven Phase auftreten kann.

Die erfindungsgemäße Kombination oder Zusammensetzung kann die Funktion der β-Zellen des Pankreas erhalten. Die erfindungsgemäße Kombination oder Zusammensetzung wird bevorzugt zur Prävention eines Funktionsverlustes der β-Zellen des Pankreas bei einem Patienten mit Diabetes, insbesondere mit einem Diabetes Typ 1 oder Typ 2 verwendet. Der Funktionsverlust der β-Zellen kann insbesondere durch Apoptose verursacht sein.

Ferner kann die erfindungsgemäße Kombination oder Zusammensetzung einen Gewichtsverlust bewirken oder/und zur Prävention einer Gewichtszunahme bei Patienten mit Diabetes, insbesondere Type I oder II, eingesetzt werden. Bei Diabetespatienten, insbesondere vom Typ 2, sind Gewichtszunahme und Übergewicht häufige Probleme. Damit kann die Verabreichung der erfindungsgemäße Kombination oder Zusammensetzung eine Therapie zur Behandlung eines Übergewichtes unterstützen.

Es versteht sich, dass die erfindungsgemäße Kombination oder Zusammensetzung eingesetzt werden kann, um bei einem Patienten mit Diabetes, insbesondere mit einem Diabetes Typ 1 oder 2, mehr als eine der hierin beschriebenen bevorzugten Indikationen zu behandeln. Daher sind von der vorliegenden Erfindung nicht nur die einzelnen bevorzugten Indikationen umfasst, sondern auch beliebige Kombinationen der Indikationen. Die erfindungsgemäße Kombination oder Zusammensetzung kann daher zur Behandlung von einer oder mehreren der hierin beschriebenen Indikationen bei Patienten mit Diabetes, insbesondere von Patienten mit Diabetes Typ 1 oder Typ 2 verwendet werden, z. B. zur Einstellung der nüchternen, der postprandialen oder/und der postabsorptiven Blutglukosekonzentration, zur Verbesserung der Glukosetoleranz, zur Prävention einer Hypoglykämie, zur Prävention eines Funktionsverlustes der β-Zellen des Pankreas, zur Gewichtsabnahme oder/und zur Prävention einer Gewichtszunahme verwendet werden. Bevorzugt ist die Einstellung der nüchternen, der postprandialen oder/und der postabsorptiven Blutglukosekonzentration, die Verbesserung der Glukosetoleranz oder/und die Prävention einer Hypoglykämie.

Die erfindungsgemäße Kombination oder Zusammensetzung kann ebenso zur Herstellung eines Medikamentes zur Behandlung von einer oder mehreren der hierin beschriebenen Indikationen verwendet werden, z. B. zur Einstellung der nüchternen, der postprandialen oder/und der postabsorptiven Blutglukosekonzentration, zur Verbesserung der Glukosetoleranz, zur Prävention einer Hypoglykämie, zur Prävention eines Funktionsverlustes der β-Zellen des Pankreas, zur Gewichtsabnahme oder/und zur Prävention einer Gewichtszunahme.

Gly(A21)-Arg(B31)-Arg(B32)-Humaninsulin und das hierin beschriebene wenigstens eine Insulin und desPro$^{36}$Exendin-4(1-39)-Lys$_6$-NH$_2$ und der hierin beschriebene wenigstens eine GLP-1-Agonist können ebenso zur Herstellung eines Medikamentes zur Behandlung von einer oder mehreren der hierin beschriebenen Indikationen verwendet werden, z. B. zur Einstellung der nüchternen, der postprandialen oder/und der postabsorptiven Blutglukosekonzentration, zur Verbesserung der Glukosetoleranz, zur Prävention einer Hypoglykämie, zur Prävention eines Funktionsverlustes der β-Zellen des Pankreas, zur Gewichtsabnahme oder/und zur Prävention einer Gewichtszunahme.

DesPro$^{36}$Exendin-4(1-39)-Lys$_6$-NH$_2$ und der hierin beschriebene wenigstens eine GLP-1-Agonist und Gly(A21)-Arg(B31)-Arg(B32)-Humaninsulin und das hierin beschriebene wenigstens eine Insulin können zusammen in einer pharmazeutischen Zusammensetzung bereitgestellt werden. Hierbei werden eine erste, eine zweite Zusammensetzung und gegebenenfalls wenigstens eine weitere pharmazeutische Zusammensetzung bereitgestellt, welche jeweils das Insulin und den GLP-1-Agonisten enthalten. Hierin beschrieben ist daher ein Arzneimittel umfassend eine erste pharmazeutische Zusammensetzung und eine zweite pharmazeutische Zusammensetzung und gegebenenfalls wenigstens eine weitere pharmazeutische Zusammensetzung, welche jeweils wenigstens ein Insulin und wenigstens einen GLP-1-Agonisten umfassen und das wenigstens eine Insulin oder/und den wenigstens einen GLP-1-Agonisten in unterschiedlichen Gewichtsanteilen bezogen auf das Gesamtgewicht der Zusammensetzung enthalten.

Hierin bedeutet "gegebenenfalls wenigstens eine weitere pharmazeutische Zusammensetzung", dass die hierin beschriebenen Arzneimittel neben der ersten und der zweiten pharmazeutischen Zusammensetzung wenigstens eine weitere pharmazeutische Zusammensetzung umfassen kann. Das hierin beschriebene Arzneimittel kann also z. B. 3, 4, 5, 6, 7, 8, 9, 10 oder mehr erfindungsgemäße pharmazeutische Zusammensetzungen umfassen.

Bevorzugt sind Arzneimittel, welche eine erste und eine zweite erfindungsgemäße pharmazeutische Zusammensetzung enthalten.

Ebenso sind Arzneimittel bevorzugt, welche eine erste, eine zweite und eine dritte erfindungsgemäße pharmazeutische Zusammensetzung enthalten.

Ebenso sind Arzneimittel bevorzugt, welche eine erste, eine zweite, eine dritte und eine vierte erfindungsgemäße pharmazeutische Zusammensetzung enthalten.

Ebenso sind Arzneimittel bevorzugt, welche eine erste, eine zweite, eine dritte, eine vierte und eine fünfte pharmazeutische Zusammensetzung enthalten.

Die Gewichtsanteile des wenigstens einen Insulins und des wenigstens einen GLP-1-Agonisten können in der ersten pharmazeutischen Zusammensetzung, der zweiten pharmazeutischen Zusammensetzung und der gegebenenfalls wenigstens einen weiteren pharmazeutischen Zusammensetzung so ausgewählt werden, dass die pharmazeutischen Zusammensetzungen unterschiedliche Verhältnisse von Insulin zum GLP-1-Agonisten bezogen auf den Gewichtsanteil enthalten.

Hierbei kann die erste Zusammensetzung das kleinste Verhältnis und die zweite Zusammensetzung das nächst größere Verhältnis enthalten. Sofern wenigstens eine weitere Zusammensetzung vorhanden ist, kann diese das nächst größere Verhältnis enthalten. Sofern noch eine weitere Zusammensetzung vorhanden ist, kann diese das wiederum nächst größere Verhältnis enthalten. Die Zusammensetzungen können also von der ersten zur zweiten und gegebenenfalls weiteren Zusammensetzungen ansteigende Verhältnisse von Insulin zum GLP-1-Agonisten bezogen auf den Gewichtsanteil enthalten.

Der Gewichtsanteil von einem der beiden Wirkstoffe, also des wenigstens einen Insulins oder des wenigstens einen GLP-1-Agonisten, in der ersten pharmazeutischen Zusammensetzung, der zweiten pharmazeutischen Zusammensetzung und der gegebenenfalls wenigstens einen weiteren pharmazeutischen Zusammensetzung wird bevorzugt jeweils so ausgewählt, dass durch Verabreichung eines bestimmten Volumens der ersten, der zweiten oder/und der wenigstens einen weiteren Zusammensetzung die vorbestimmte Dosis dieses Wirkstoffs verabreicht werden kann. Besonders bevorzugt ist dieser Wirkstoff das wenigstens eine Insulin.

Der Gewichtsanteil des anderen der beiden Wirkstoffe, also des wenigstens einen Insulins oder des wenigstens einen GLP-1-Agonisten, in der ersten pharmazeutischen Zusammensetzung, der zweiten pharmazeutischen Zusammensetzung und der gegebenenfalls wenigstens einen weiteren pharmazeutischen Zusammensetzung wird bevorzugt so ausgewählt, dass die Verhältnisse von Insulin zum GLP-1-Agonisten bezogen auf den Gewichtsanteil von der ersten zur zweiten und gegebenenfalls weiteren Zusammensetzungen ansteigen. Besonders bevorzugt ist dieser Wirkstoff der wenigstens eine GLP-1-Agonist.

Darüber hinaus wird der Gewichtsanteil des anderen der beiden Wirkstoffe in den pharmazeutischen Zusammensetzungen so bestimmt, dass eine der pharmazeutischen Zusammensetzungen so ausgewählt werden kann, dass die zu verabreichende Dosis des ersten der beiden Wirkstoffe und die zu verabreichende Dosis des zweiten Wirkstoffs in einem bestimmten Volumen gegeben sind. Damit wird eine pharmazeutische Zusammensetzung ausgewählt, welche das gewünschte Verhältnis enthält.

Theoretisch könnte für jedes einzelne therapeutisch gewünschte Verhältnis der Gewichtsanteile vom wenigstens einen Insulin zum wenigstens einen GLP-1-Agonisten eine pharmazeutische Zusammensetzung bereitgestellt werden, um eine optimale bedarfsgerechte Dosierung für beide Wirkstoffe für jeden Patienten zu erzielen.

Es ist eine bestimmte Anzahl von pharmazeutischen Zusammensetzungen ausreichend, um die in der Praxis notwendigen Dosierungen für beide Wirkstoffe zu erfassen. Es wird für jeden Patienten ein bestimmter Dosierungsbereich innerhalb eines therapeutisch sinnvollen Intervalls für jeden der beiden Wirkstoffe bestimmt. Die zu verabreichende Dosis soll hierbei für einen bestimmten Patienten im wesentlichen innerhalb dieses Dosierungsbereiches schwanken, ohne dass eine Über- oder eine Unterdosierung vorliegt.

Überraschenderweise wurde gefunden, dass die synergisten Wirkungen der Kombination eines Insulins (Gly(A21)-Arg(B31)-Arg(B32)-Humaninsulin) und eines GLP-1-Agonisten (desPro$^{36}$Exendin-4(1-39)-Lys$_6$-NH$_2$) auf die Glukosekonzentration im Blutplasma in einem Konzentrationsbereich des GLP-1-Agonisten von einer Größenordnung (Faktor 10) auftreten. Da primär die Insulinmenge an den einzelnen Patienten angepasst und präzise dosiert werden muss, ermöglicht es der synergistische Konzentrationsbereich vom GLP-1-Agonisten, dass eine erfindungsgemäße pharmazeutische Zusammensetzung, welche ein bestimmtes Verhältnis von wenigstens einem Insulin zu dem wenigstens einem GLP-1-Agonisten enthält, einen therapeutischen Bereich von Insulindosierungen gleichzeitig mit der zugehörigen synergistischen GLP-1-Agonistenmenge abdeckt. Das Verhältnis kann so ausgewählt werden, dass zu jeder gewünschten Insulindosierung eine Dosierung des wenigstens einen GLP-1-Agonisten korrespondiert, welche innerhalb des gewünschten Bereichs, z.B. des synergistischen Bereichs, liegt. Wie weiter oben ausgeführt, können die Verhältnisse der ersten, zweiten und gegebenenfalls wenigstens einen weiteren Zusammensetzung des Arzneimittels ferner so gewählt werden, dass die Verhältnisse von der ersten zur zweiten und der gegebenenfalls wenigstens einen weiteren Zusammensetzung ansteigen. Ist die Dosierung des GLP-1-Agonisten bei der gewünschten Insulindosierung einer Zusammensetzung (z. B. der ersten Zusammensetzung) außerhalb (in der Regel oberhalb) des gewünschten Dosierungsbereiches des GLP-1-Agonisten, so wird die nächste Zusammensetzung (z.B. die zweite Zusammensetzung) oder eine weitere Zusammensetzung mit einem größeren Verhältnis des wenigstens einen Insulins zu dem wenigstens einen GLP-1-Agonisten zur Anwendung ausgewählt, in welcher die Menge des GLP-1-Agonisten bei der gewünschten Insulindosis im gewünschten Bereich liegt. Die Verhältnisse der ersten, zweiten und gegebenenfalls wenigstens einen weiteren Zusammensetzung des Arzneimittels können ferner so gewählt werden, dass die Bereiche der Insulindosierungen, welche zu den gewünschten Dosierungen des wenigstens einen GLP-1-Agonisten korrespondieren, aneinander anschließen oder/und einander überlappen. Bevorzugt überlappen die Bereiche. Überlappung bedeutet insbesondere, dass wenigstens zwei Zusammensetzungen ausgewählt werden können, welche bei der gewünschten Dosierung des wenigstens einen Insulins je eine Menge des wenigstens einen GLP-1-Agonisten enthalten, welche innerhalb des gewünschten Dosierungsbereichs liegt.

Beispielsweise genügen 3 Zusammensetzungen, um die Dosis des wenigstens einen Insulins für einen individuellen Patienten auf einen Wert ausgewählt aus dem Bereich von 15 bis 80 Einheiten Insulin einzustellen und gleichzeitig den GLP-1-Agonisten mit einer Menge innerhalb des Bereichs von 10 bis 20 µg zu dosieren (siehe Beispiel 11).

Ebenso wird hierin ein Arzneimittel bereitgestellt, in welchem das Verhältnis so ausgewählt wird, dass zu jeder gewünschten Dosierung des GLP-1-Agonisten eine Dosierung des wenigstens einen Insulins korrespondiert, welche innerhalb des gewünschten Bereichs, z.B. des synergistischen Bereichs, liegt. Die Verhältnisse der ersten, zweiten und gegebenenfalls wenigstens einen weiteren Zusammensetzung des Arzneimittels können ferner so gewählt werden, dass die Bereiche der Dosierungen des GLP-1-Agonisten, welche zu den gewünschten Dosierungen des wenigstens einen Insulins korrespondieren, aneinander anschließen oder/und einander überlappen. Bevorzugt überlappen die Bereiche. Überlappung bedeutet in diesem Zusammenhang insbesondere, dass wenigstens zwei Zusammensetzungen ausgewählt werden können, welche bei der gewünschten Dosierung des wenigstens einen GLP-1-Agonisten je eine Menge des wenigstens einen Insulins enthalten, welche innerhalb des gewünschten Dosierungsbereichs liegt.

Vorzugsweise enthält das hierin beschriebene Arzneimittel maximal 10 pharmazeutische Zusammensetzungen wie oben definiert, stärker bevorzugt maximal 5, maximal 4, maximal 3 oder 2 pharmazeutische Zusammensetzungen. Die hierin beschriebenen Zusammensetzungen können das wenigstens eine Insulin in jeweils identischen oder verschiedenen Gewichtsanteilen enthalten. Beispielsweise können mindestens zwei der hierin beschriebenen Zusammensetzungen das wenigstens eine Insulin in einem im wesentlichen identischen Gewichtsanteil enthalten.

Es ist bevorzugt, dass die erste, zweite und die gegebenenfalls weitere Zusammensetzung das wenigstens eine Insulin in einem im wesentlichen identischen Gewichtsanteil enthalten und den wenigstens einen GLP-1-Agonisten in unterschiedlichen Gewichtsanteilen enthalten.

Die hierin beschriebenen Zusammensetzungen können den wenigstens einen GLP-1-Agonisten in jeweils identischen oder verschiedenen Gewichtsanteilen enthalten. Beispielsweise können mindestens zwei der hierin beschriebenen Zusammensetzungen den wenigstens einen GLP-1-Agonisten in einem im wesentlichen identischen Gewichtsanteil enthalten.

Es ist ebenso bevorzugt, dass die erste, zweite und die gegebenenfalls weitere Zusammensetzung den wenigstens einen GLP-1-Agonisten in einem im wesentlichen identischen Gewichtsanteil enthalten und das wenigstens eine Insulin in unterschiedlichen Gewichtsanteilen enthalten.

Neben den ersten, zweiten und der gegebenenfalls wenigstens einen weiteren Zusammensetzung kann das hierin beschriebene Arzneimittel wenigstens eine weitere pharmazeutische Zusammensetzung enthalten, welche entweder wenigstens ein Insulin oder wenigstens einen GLP-1-Agonisten enthält. Ebenso kann das hierin beschriebene Arzneimittel wenigstens eine weitere pharmazeutische Zusammensetzung enthalten, welche wenigstens ein Insulin und wenigstens einen GLP-1-Agonisten in einem Verhältnis der Gewichtsanteile wie die hierin beschriebene erste, zweite oder gegebenenfalls weitere pharmazeutische Zusammensetzung enthält.

Weiterhin ist hierin ein Arzneimittel beschrieben, umfassend eine erste pharmazeutische Zusammensetzung und eine zweite pharmazeutische Zusammensetzung, wobei die erste pharmazeutische Zusammensetzung wenigstens ein Insulin umfasst und die zweite pharmazeutische Zusammensetzung wenigstens einen GLP-1-Agonisten umfasst und wobei das Arzneimittel zur unabhängigen Verabreichung der ersten und zweiten pharmazeutischen Zusammensetzung formuliert oder/und konfektioniert ist.

Das Beispiel 12 zeigt, auf welche Weise eine Kombination von zwei oder mehr Wirkstoffen so formuliert werden kann, dass bei Kombination von zwei oder mehr Zusammensetzungen beide Wirkstoffe in beliebigen Mengen und in beliebigen Verhältnissen zueinander verabreicht werden können. Hierbei wird berücksichtigt, dass mindestens einer der Wirkstoffe durch die Kombination (z.B. durch Mischen direkt vor der Verabreichung) nicht verdünnt werden darf.

Hierin wird ein Arzneimittel bereitgestellt, welches einen ersten Wirkstoff und einen zweiten Wirkstoff und gegebenenfalls mindestens einen weiteren Wirkstoff umfasst, wobei diese Wirkstoffe in einer ersten, einer zweiten und gegebenenfalls mindestens einer weiteren Zusammensetzung bereitgestellt werden. Der erste Wirkstoff ist in allen Zusammensetzungen enthalten. Der zweite Wirkstoff ist in der zweiten Formulierung enthalten, und der gegebenenfalls mindestens eine weitere Wirkstoff ist in der optionalen mindesten einen weiteren Zusammensetzung enthalten. Damit enthalten die zweite und jede weitere Zusammensetzung den ersten Wirkstoff kombiniert mit einem anderen Wirkstoff.

Damit ist hierin ein Arzneimittel beschrieben, welches eine erste pharmazeutische Zusammensetzung und eine zweite pharmazeutische Zusammensetzung und gegebenenfalls wenigstens eine weitere pharmazeutische Zusammensetzung umfasst, wobei die erste pharmazeutische Zusammensetzung wenigstens einen ersten Wirkstoff umfasst, und wobei die zweite pharmazeutische Zusammensetzung wenigstens einen ersten Wirkstoff und wenigstens einen zweiten Wirkstoff umfasst, und wobei die wenigstens eine weitere pharmazeutische Zusammensetzung wenigstens einen ersten und wenigstens einen weiteren Wirkstoff umfasst. Hierbei können die Wirkstoffe beliebige Wirkstoffe sein.

Vorzugsweise enthält die erste Zusammensetzung als Wirkstoff nur den wenigstens einen ersten Wirkstoff.

Die erste, zweite und die gegebenenfalls wenigstens eine weitere Zusammensetzung können den ersten Wirkstoff in einem im wesentlichen gleichen Gewichtsanteil oder in unterschiedlichen Gewichtsanteilen bezogen auf das Gesamtgewicht der Zusammensetzung enthalten.

Es ist bevorzugt, dass die erste pharmazeutische Zusammensetzung, die zweite pharmazeutische Zusammensetzung und die gegebenenfalls wenigstens eine weitere pharmazeutische Zusammensetzung den ersten Wirkstoff in im wesentlichen gleichen Gewichtsanteilen bezogen auf das Gesamtgewicht der Zusammensetzung enthalten. Hierdurch kann erreicht werden, dass jedes beliebige Verhältnis der ersten und der zweiten und gegebenenfalls jedes beliebige Verhältnis der ersten und der wenigstens einen weiteren Zusammensetzung eingesetzt werden kann, wobei die Dosierung des ersten Wirkstoffs über die Gesamtmenge der verabreichten Zusammensetzungen erfolgt. Über das Verhältnis der beiden Zusammensetzungen lässt sich die Menge des Wirkstoffs, welcher nur in der zweiten und gegebenenfalls der wenigstens einen weiteren Zusammensetzung enthalten ist, stufenlos hinzudosieren. Damit kann also ohne weiteres jede gewünschte Menge und jedes gewünschte Verhältnis vom ersten zum zweiten Wirkstoff und gegebenenfalls vom ersten Wirkstoff zu einem weiteren Wirkstoff dosiert werden, ohne dass sich die Konzentration des ersten Wirkstoffs ändert.

Der erste Wirkstoff kann wenigstens ein Insulin sein. Der zweite Wirkstoff kann wenigstens ein GLP-1-Agonist sein. Bevorzugt ist ein Arzneimittel, welches eine erste pharmazeutische Zusammensetzung und eine zweite pharmazeutische Zusammensetzung und gegebenenfalls wenigstens eine weitere pharmazeutische Zusammensetzung umfasst, wobei die erste pharmazeutische Zusammensetzung wenigstens ein Insulin umfasst, und wobei die zweite pharmazeutische Zusammensetzung wenigstens ein Insulin und wenigstens einen GLP-1-Agonisten umfasst, und wobei die wenigstens eine weitere pharmazeutische Zusammensetzung wenigstens ein Insulin und wenigstens einen weiteren Wirkstoff umfasst.

Vorzugsweise enthält die erste Zusammensetzung als Wirkstoff nur das wenigstens eine Insulin.

Der weitere Wirkstoff kann ein beliebiger Wirkstoff sein. Insbesondere ist der weitere Wirkstoff ein Wirkstoff, welcher zur Behandlung von Patienten mit Diabetes mellitus (Typ 1 oder/und Typ 2) eingesetzt wird, wobei auch Wirkstoffe zur Behandlung von Begleiterkrankungen von Diabetes mit eingeschlossen sind.

Die erste, zweite und die gegebenenfalls wenigstens eine weitere Zusammensetzung können das Insulin in einem im wesentlichen gleichen Gewichtsanteil oder in unterschiedlichen Gewichtsanteilen bezogen auf das Gesamtgewicht der Zusammensetzung enthalten.

Es ist bevorzugt, dass die erste pharmazeutische Zusammensetzung, die zweite pharmazeutische Zusammensetzung und die gegebenenfalls wenigstens eine weitere pharmazeutische Zusammensetzung das Insulin in im wesentlichen gleichen Gewichtsanteilen bezogen auf das Gesamtgewicht der Zusammensetzung enthalten. Hierdurch kann erreicht werden, dass jedes beliebige Verhältnis der ersten und der zweiten und gegebenenfalls jedes beliebige Verhältnis der erster und der wenigstens einen weiteren Zusammensetzung eingesetzt werden kann, wobei die Dosierung des Insulins über die Gesamtmenge der verabreichten Zusammensetzungen erfolgt. Über das Verhältnis der beiden Zusammensetzungen lässt sich die Menge des Wirkstoffs, welcher nur in der zweiten und gegebenenfalls der wenigstens einen weiteren Zusammensetzung enthalten ist, stufenlos hinzudosieren. Damit kann also ohne weiteres jede gewünschte Menge und jedes gewünschte Verhältnis von Insulin zu GLP-1-Agonist und gegebenenfalls von Insulin zu einem weiteren Wirkstoff dosiert werden, ohne dass sich die Konzentration des wenigstens einen Insulins ändert.

Hierin ist unter "im wesentlichen gleichen Gewichtsanteilen" eines Wirkstoffs in zwei Zusammensetzungen zu verstehen, dass eine der beiden Zusammensetzungen den Wirkstoff in einem Gewichtsanteil enthält, welcher z.B. nicht mehr als 10%, nicht mehr als 5%, nicht mehr als 1% oder nicht mehr als 0,1% höher ist als sein Gewichtsanteil in der anderen Zusammensetzung.

Ebenso kann der erste Wirkstoff wenigstens ein GLP-1-Agonist sein. Der zweite Wirkstoff kann wenigstens ein Insulin sein. Bevorzugt ist ein Arzneimittel, welches eine erste pharmazeutische Zusammensetzung und eine zweite pharmazeutische Zusammensetzung und gegebenenfalls wenigstens eine weitere pharmazeutische Zusammensetzung umfasst, wobei die erste pharmazeutische Zusammensetzung wenigstens einen GLP-1-Agonisten umfasst, und wobei die zweite pharmazeutische Zusammensetzung wenigstens einen GLP-1-Agonisten und wenigstens ein Insulin umfasst, und wobei die wenigstens eine weitere pharmazeutische Zusammensetzung wenigstens einen GLP-1-Agonisten und wenigstens einen weiteren Wirkstoff umfasst.

Vorzugsweise enthält die erste Zusammensetzung als Wirkstoff nur den wenigstens einen GLP-1-Agonisten.

Die erste, zweite und die gegebenenfalls wenigstens eine weitere Zusammensetzung können den GLP-1-Agonisten in einem im wesentlichen gleichen Gewichtsanteil oder in unterschiedlichen Gewichtsanteilen bezogen auf das Gesamtgewicht der Zusammensetzung enthalten. Es ist bevorzugt, dass die erste pharmazeutische Zusammensetzung, die zweite pharmazeutische Zusammensetzung und die gegebenenfalls wenigstens eine weitere pharmazeutische Zusammensetzung den wenigstens einen GLP-1-Agonisten in im wesentlichen gleichen Gewichtsanteilen bezogen auf das Gesamtgewicht der Zusammensetzung enthalten.

Das hierin beschriebene Arzneimittel weist gegenüber Zusammensetzungen des Standes der Technik, welche aus getrennten Zusammensetzungen mit jeweils einem Wirkstoff, insbesondere mit einem Insulin bzw. einem GLP-1-Agonisten, mehrere Vorteile auf:

- Das Verhältnis des ersten Wirkstoffs zum zweiten Wirkstoff und gegebenenfalls des ersten Wirkstoffs zum wenigstens einen weiteren Wirkstoff kann durch den Verwender frei gewählt werden.
- Da der erste Wirkstoff in allen Zusammensetzungen vorhanden ist, insbesondere in gleichen Gewichtsanteilen, wird dieser Wirkstoff nicht verdünnt, wenn die erste Zusammensetzung mit der zweiten und gegebenenfalls weiteren Zusammensetzungen gemischt wird. Dies ist wichtig für Wirkstoffe wie z.B. Insulin, bei denen die Pharmakokinetik wird durch die Konzentration/Verdünnung beeinflusst wird.
- Das Injektionsvolumen wird verkleinert (siehe Beispiel 12). Damit verringert sich die Verdünnung des zweiten Wirkstoffs (z.B. eines GLP-1-Agonisten) und gegebenenfalls eines weiteren Wirkstoffs.

Ein weiterer erfindungsgemäßer Gegenstand ist ein Kit umfassend eine erfindungsgemäße Kombination oder Zusammensetzung. Der erfindungsgemäße Kit kann für die Anwendung durch medizinisches Personal oder durch medizinische Laien, insbesondere den Patienten selbst oder Hilfspersonen wie Angehörige bestimmt sein. Außerdem ist hierin ein Kit beschrieben, in welchem die einzelnen pharmazeutischen Zusammensetzungen des hierin beschriebenen Arzneimittels in getrennten Verpackungen zusammengefasst sind, so dass der Patient die jeweils an den aktuellen Bedarf angepasste Zusammensetzung auswählen und eine bedarfsgerechte Menge verabreichen kann. Der hierin beschriebene Kit umfasst zum Beispiel das hierin beschriebene Arzneimittel in Form eines Satzes von Spritzen, Glasampullen oder/und Stiften, welche eine erfindungsgemäße Zusammensetzung enthalten.

Die erfindungsgemäße Kombination oder Zusammensetzung kann auf verschiedenen Wegen verabreicht werden. Die erfindungsgemäße Kombination oder Zusammensetzung kann parenteral verabreicht werden. Die Kombination oder Zusammensetzung kann injiziert werden, wobei Injektionssysteme mit oder ohne Injektionsnadel verwendet werden können. Ferner kann die Kombination oder Zusammensetzung per Inhalation verabreicht werden. Hierbei können flüssige Zusammensetzungen inhaliert werden, ober die Zusammensetzungen können in Form von Pulver inhaliert werden. Außerdem kann die erfindungsgemäße Kombination oder Zusammensetzung per Spray, insbesondere Nasenspray verabreicht werden. Darüber hinaus kann die erfindungsgemäße Kombination oder Zusammensetzung durch ein transdermales System verabreicht werden. Der Fachmann kennt diese Verabreichungsmethoden und kann die erfindungsgemäße Kombination oder Zusammensetzung so formulieren, dass es durch eines dieser Verabreichungsmethoden wirksam verabreicht werden kann. Es ist bevorzugt, dass die erfindungsgemäßen Zusammensetzungen flüssig sind. Ferner ist es ist bevorzugt, dass die erfindungsgemäße Kombination oder Zusammensetzung parenteral verabreicht, insbesondere injiziert wird.

Ein weiterer Gegenstand der vorliegenden Erfindung ist eine Vorrichtung zur Verabreichung der erfindungsgemäßen Kombination oder Zusammensetzung. Die erfindungsgemäße Vorrichtung kann eine Vorrichtung zur parenteralen Verabreichung sein. Die erfindungsgemäße Vorrichtung kann eine Vorrichtung zur Injektion mit oder ohne Injektionsnadel ein. Ferner kann die Vorrichtung eine Vorrichtung zur Inhalation sein, wobei flüssige Zusammensetzungen inhaliert werden, ober die Zusammensetzungen können in Form von Pulver inhaliert werden. Außerdem kann die Vorrichtung eine Vorrichtung zur Verabreichung eines Sprays, insbesondere eines Nasensprays sein. Darüber hinaus kann die Vorrichtung ein transdermales Verabreichungssystem sein. Es ist bevorzugt, dass die erfindungsgemäße Vorrichtung eine Vorrichtung zur parenteralen Verabreichung, insbesondere eine Injektionsvorrichtung ist.

"Konfektionierung" ist ein Begriff, welcher dem Fachmann bekannt ist und in der Pharmakologie die Endbehandlung, z. B. Portionierung und Verpackung, von Arzneimitteln zur Verwendung durch den Endverbraucher bezeichnet. In der vorliegenden Anmeldung bedeutet "konfektioniert" oder "Konfektionierung" insbesondere, dass die erfindungsgemäßen pharmazeutischen Zusammensetzungen in einer therapeutisch wirksamen Menge auf geeignete Weise verpackt sind, um die hierin beschriebene Auswahl von mindestens einer der Zusammensetzungen des hierin beschriebenen Arzneimittels zur gewünschten Dosierung des wenigstens einen Insulins und des wenigstens einen GLP-1-Agonisten zu ermöglichen. Insbesondere ist eine parenterale Verabreichung, bevorzugt eine Injektion, stärker bevorzugt zur subkutanen Injektion, vorgesehen. Eine geeignete Verpackung ist z. B. eine Spritze oder ein Glasgefäß mit einem geeigneten Verschluß, aus denen bei Bedarf einzelne therapeutisch wirksame Dosen entnommen werden können. Ebenso geeignet sind Injektionsstifte ("Stifte", "Pens") zur Verabreichung von Insulin, welche einen Behälter (z. B. eine Patrone) umfassen, welcher eine erfindungsgemäße pharmazeutische Zusammensetzung enthält.

"Formulieren" oder "Formulierung" ist ein Begriff, welcher dem Fachmann bekannt ist und auf dem Gebiet der Pharmakologie die Herstellung von Arzneimitteln und Arzneimittelzusammensetzungen und die Zubereitung mit Hilfsstoffen bezeichnet. In der vorliegenden Anmeldung bedeutet "formulieren" oder "Formulierung" insbesondere, dass die erfindungsgemäße Zusammensetzung in einer geeigneten Form bereitgestellt wird, welche eine Verabreichung einer therapeutisch wirksamen Menge der Wirkstoffe ermöglicht. Insbesondere ist eine Formulierung zur parenteralen Verabreichung, bevorzugt zur Injektion, stärker bevorzugt zur subkutanen Injektion, vorgesehen.

Hierin schließt der Begriff "GLP-1-Agonist" GLP-1, Analoga und Derivate davon, Exendin-3 und Analoga und Derivate davon, Exendin-4 und Analoga und Derivate davon ein. Die erfindungsgemäßen Zusammensetzungen umfassen eines oder mehrere unabhängig voneinander ausgewählt aus der Gruppe bestehend aus Glucagon-Like Peptide-1 (GLP-1), Analoga und Derivaten von GLP-1, Exendin-3, Analoga und Derivaten von Exendin-3, Exendin-4, Analoga und Derivaten von Exendin-4 und pharmakologisch tolerierbaren Salzen davon. Ferner sind Substanzen eingeschlossen, welche die biologische Aktivität von GLP-1 aufweisen.

GLP-1 Analoga und Derivate sind z.B. in der WO 98/08871 beschrieben, Exendin-3, Analoga und Derivate von Exendin-3, Exendin-4 und Analoga und Derivate von Exendin-4 können in WO 01/04156, WO 98/30231, US 5,424,286, in der EP-Anmeldung 99 610043.4, in WO 2004/005342 und WO 04/035623 gefunden werden. Diese Dokumente sind hierin durch Bezugnahme eingeschlossen ist. Das in diesen Dokumenten beschriebene Exendin-3, Exendin-4 und die dort beschriebenen Analoga und Derivate davon können in den Zusammensetzungen der vorliegenden Erfindung als GLP-1-Agonisten verwendet werden. Ebenso können beliebige Kombinationen der in diesen Dokumenten beschriebenen Exendin-3, Exendin-4 und der dort beschriebenen Analoga und Derivate als GLP-1-Agonisten verwendet werden. Der wenigstens eine GLP-1-Agonist ist vorzugsweise unabhängig ausgewählt aus der Gruppe bestehend aus Exendin-4, Analoga und Derivaten von Exendin-4 und pharmakologisch tolerierbaren Salzen davon.

Ein weiterer bevorzugter GLP-1-Agonist ist ein Analogon von Exendin-4 ausgewählt aus einer Gruppe bestehend aus:

H-desPro$^{36}$-Exendin-4-Lys$_6$-NH$_2$,
H-des(Pro$^{16,37}$)-Exendin-4-Lys$_4$-NH$_2$,
H-des(Pro$^{36,37}$)-Exendin-4-Lys$_5$-NH$_2$ und pharmakologisch tolerierbaren Salzen davon.

Ein weiterer bevorzugter GLP-1-Agonist ist ein Analogon von Exendin-4 ausgewählt aus einer Gruppe bestehend aus:

desPro$^{36}$[Asp$^{28}$]Exendin-4 (1-39),
desPro$^{36}$[IsoAsp$^{28}$]Exendin-4 (1-39),
desPro$^{36}$[Met(O)$^{14}$, Asp$^{28}$]Exendin-4 (1-39),
desPro$^{36}$[Met(O)$^{14}$, IsoAsp$^{28}$]Exendin-4 (1-39),
desPro$^{36}$ [Trp(O$_2$)$^{25}$, Asp$^{28}$]Exendin-2 (1-39),
desPro$^{36}$ [Trp(O$_2$)$^{25}$, IsoAsp$^{28}$]Exendin-2 (1-39),
desPro$^{36}$ [Met(O)$^{14}$Trp(O$_2$)$^{25}$, Asp$^{28}$]Exendin-4 (1-39),
desPro$^{36}$ [Met(O)$^{14}$Trp(O$_2$)$^{25}$, IsoAsp$^{28}$]Exendin-4 (1-39) und pharmakologisch tolerierbaren Salzen davon.

Ein weiterer bevorzugter GLP-1-Agonist ist ein Analogon von Exendin-4 ausgewählt aus einer Gruppe wie im vorigen Absatz beschrieben, bei denen an die C-Termini der Analoga von Exendin-4 das Peptid -Lys$_6$-NH$_2$ angefügt ist.

Ein weiterer bevorzugter GLP-1-Agonist ist ein Analogon von Exendin-4 ausgewählt aus einer Gruppe bestehend aus:

H-(Lys)$_6$- des Pro$^{36}$ [Asp$^{28}$]Exendin-4(1-39)-Lys$_6$-NH$_2$
des Asp$^{28}$Pro$^{36}$, Pro$^{37}$, Pro$_{38}$ Exendin-4(1-39) -NH$_2$,
H-(Lys)$_6$- des Pro$^{36}$, Pro$^{37}$, Pro$^{38}$ [Asp$^{28}$]Exendin-4(1-39) -NH$_2$,
H-Asn-(Glu)$_5$ des Pro$^{36}$, Pro$^{37}$, Pro$^{38}$ [Asp$^{28}$]Exendin-4(1-39) -NH$_2$,
des Pro$^{36}$, Pro$^{37}$, Pro$^{38}$ [Asp$^{28}$]Exendin-4(1-39)-(Lys)$_6$-NH$_2$,
H-(Lys)$_6$- des Pro$^{36}$, Pro$^{37}$, Pro$^{38}$ [Asp$^{28}$]Exendin-4(1-39)-(Lys)$_6$-NH$_2$,
H-Asn-(Glu)$_5$- des Pro$^{36}$, Pro$^{37}$, Pro$^{38}$ [Asp$^{28}$]Exendin-4(1-39)-(Lys)$_6$-NH$_2$,
H-(Lys)$_6$- des Pro$^{36}$ [Trp(O$_2$)$^{25}$, Asp$^{28}$]Exendin-4(1-39)-Lys$_6$-NH$_2$,
H- des Asp$^{28}$ Pro$^{36}$, Pro$^{37}$, Pro$^{38}$ [Trp(O$_2$)$^{25}$]Exendin-4(1-39) -NH$_2$,
H-(Lys)$_6$- des Pro$^{36}$, Pro$^{37}$, Pro$^{38}$ [Trp(O$_2$)$^{25}$, Asp$^{28}$]Exendin-4(1-39) -NH$_2$,
H-Asn-(Glu)$_5$- des Pro$^{36}$, Pro$^{37}$, Pro$^{38}$ [Trp(O$_2$)$^{25}$, Asp$^{28}$]Exendin-4(1-39) -NH$_2$,
des Pro$^{36}$, Pro$^{37}$, Pro$^{38}$ [Trp(O$_2$)$^{25}$, Asp$^{28}$]Exendin-4(1-39)-(Lys)$_6$-NH$_2$,
H-(Lys)$_6$- des Pro$^{36}$, Pro$^{37}$, Pro$^{38}$ [Trp(O$_2$)$^{25}$, Asp$^{28}$]Exendin-4(1-39)-(Lys)$_6$-NH$_2$,
H-Asn-(Glu)$_5$- des Pro$^{36}$, Pro$^{37}$, Pro$^{38}$ [Trp(O$_2$)$^{25}$, Asp$^{28}$]Exendin-4(1-39)-(Lys)$_6$-NH$_2$,
H-(Lys)$_6$- des Pro$^{36}$ [Met(O)$^{14}$, Asp$^{28}$]Exendin-4(1-39)-Lys$_6$-NH$_2$,
des Met(O)$^{14}$ Asp$^{28}$ Pro $^{36}$, Pro$^{37}$, Pro$^{38}$ Exendin-4(1-39) -NH$_2$,
H-(Lys)$_6$- des Pro$^{36}$, Pro $^{37}$, Pro$^{38}$ [Met(O)$^{14}$, Asp$^{28}$]Exendin-4(1-39) -NH$_2$,
H-Asn-(Glu)$_5$- des Pro$^{36}$, Pro$^{37}$, Pro$^{38}$ [Met(O)$^{14}$, Asp$^{28}$] Exendin-4(1-39) -NH$_2$,
des Pro$^{36}$, Pro$^{37}$, Pro$^{38}$ [Met(O)$^{14}$, Asp$^{28}$]Exendin-4(1-39)-(Lys)$_6$-NH$_2$,
H-(Lys)$_6$- des Pro$^{36}$, Pro$^{37}$, Pro$^{38}$ [Met(O)$^{14}$, Asp$^{28}$]Exendin-4(1-39)-Lys$_6$-NH$_2$,
H-Asn-(Glu)$_5$ des Pro$^{36}$, Pro$^{37}$, Pro$^{38}$ [Met(O)$^{14}$, Asp$^{28}$] Exendin-4(1-39)-(Lys)$_6$-NH$_2$,

H-(Lys)$_6$- des Pro$^{36}$ [Met(O)$^{14}$, Trp(O$_2$)$^{25}$, Asp$^{28}$]Exendin-4(1-39)-Lys$_6$-NH$_2$,

des Asp$^{28}$ Pro$^{36}$, Pro$^{37}$, Pro$^{38}$ [Met(O)$^{14}$, Trp(O$_2$)$^{25}$]Exendin-4(1-39) -NH$_2$,

H-(Lys)$_6$- des Pro$^{36}$' Pro$^{37}$, Pro$^{38}$ [Met(O)$^{14}$, Trp(O$_2$)$^{25}$, Asp$^{28}$]Exendin-4(1-39) -NH$_2$,

H-Asn-(Glu)5- des Pro$^{36}$, Pro$^{37}$, Pro$^{38}$ [Met(O)$^{14}$, Asp$^{28}$] Exendin-4(1-39) -NH$_2$,

des Pro$^{36}$, Pro$^{37}$, Pro$^{38}$ [Met(O)$^{14}$, Trp(O$_2$)$^{25}$, Asp$^{28}$]Exendin-4(1-39)-(Lys)$_6$-NH$_2$,

H-(Lys)$_6$- des Pro$^{36}$, Pro$^{37}$, Pro$^{38}$ [Met(O)$^{14}$, Trp(O$_2$)$^{25}$, Asp$^{28}$]Exendin-4(1-39)-(Lys)$_6$-NH$_2$,

H-Asn-(Glu)$_5$- des Pro$^{36}$, Pro$^{37}$, Pro$^{38}$ [Met(O)$^{14}$, Trp(O$_2$)$^{25}$, Asp$^{28}$] Exendin-4(1-39)-(Lys)$_6$-NH$_2$ und pharmakologisch tolerierbaren Salzen davon.

Ein weiterer bevorzugter GLP-1-Agonist ist ausgewählt aus einer Gruppe bestehend aus Arg$^{34}$,Lys$^{26}$(N$^\epsilon$($\gamma$-glutamyl(N$^\alpha$-hexadecanoyl)))GLP-1(7-37) [Liraglutide] und einem pharmakologisch tolerierbaren Salz davon.

Ein weiterer bevorzugter GLP-1-Agonist ist AVE0010. AVE0010 weist die Sequenz: des Pro$^{36}$Exendin-4(1-39)-Lys$_6$-NH$_2$

auf. Diese Substanz ist als SEQ ID NO:93 in WO 01/04156 veröffentlicht. Ebenso sind pharmakologisch tolerierbare Salze von AVE0010 bevorzugt.

Der Begriff "wenigstens ein GLP-1-Agonist" schließt Kombinationen der hierin beschriebenen GLP-1-Agonisten ein, welche in den erfindungsgemäßen Zusammensetzungen verwendet werden, z. B. beliebige Kombinationen von zwei oder mehr GLP-1-Agonisten ausgewählt aus den hierin beschriebenen GLP-1-Agonisten.

Der wenigstens eine GLP-1-Agonist ist weiterhin vorzugsweise unabhängig ausgewählt aus Exendin-4, des Pro$^{36}$Exendin-4(1-39)-Lys$_6$-NH$_2$ und Arg$^{34}$,Lys$^{26}$(N$^\epsilon$($\gamma$-glutamyl(N$^\alpha$-hexadecanoyl)))GLP-1(7-37) [Liraglutide] und pharmakologisch tolerierbaren Salzen davon.

Die hierin beschriebenen Zusammensetzungen enthalten den GLP-1-Agonisten in einer Menge von 10 $\mu$g/ml bis 20 mg/ml, bevorzugt 25 $\mu$g/ml bis 15 mg/ml. Für die sauren bis neutral gelösten GLP-1 Agonisten gilt bevorzugt 20 $\mu$g/ml bis 300 $\mu$g/ml und für die neutral bis basischen bevorzugt 500 $\mu$g/ml bis 10 mg/ml. Für Exendin-4 Analoge sind 20 $\mu$g/ml bis 150 $\mu$g/ml bevorzugt.

Wie hierin beschrieben, umfasst der Begriff "Insulin" nicht nur unmodifizierte Insuline, sondern auch Insulinanaloga, Insulinderivate und Insulinmetabolite. Die hierin beschriebenen Zusammensetzungen umfassen eines oder mehrere unabhängig ausgewählt aus der Gruppe bestehend aus Insulinen (z.B. unmodifizierten Insulinen), Insulinanaloga, Insulinderivaten und Insulinmetaboliten und beliebigen Kombinationen davon.

Das wenigstens eine Insulin kann unabhängig ausgewählt sein aus der Gruppe bestehend aus Rinderinsulinen, Analoga, Derivaten und Metaboliten davon, Schweineinsulinen, Analoga, Derivaten und Metaboliten davon und Humaninsulinen, Analoga, Derivaten und Metaboliten davon. Bevorzugt ist das wenigstens eine Insulin unabhängig ausgewählt aus Humaninsulinen, Analoga, Derivaten und Metaboliten davon.

Ferner kann ein hierin beschriebenes Insulin unabhängig aus unmodifizierten Insulinen ausgewählt sein, insbesondere aus Rinderinsulinen, Schweineinsulinen und Humaninsulinen.

Das wenigstens eine Insulin kann unabhängig ausgewählt sein aus der Gruppe bestehend aus Rinderinsulinen, Schweineinsulinen und Humaninsulinen. Stärker bevorzugt ist das wenigstens eine Insulin unabhängig ausgewählt aus Humaninsulinen. Ein hierin beschriebenes Insulin kann aus unmodifizierten Insulinen ausgewählt sein, insbesondere aus Rinderinsulinen, Schweineinsulinen und Humaninsulinen.

Hierin beschriebene Insulinderivate sind Derivate von einem natürlich vorkommenden Insulin oder/und einem Insulinanalogon, welche durch chemische Modifizierung erhalten werden. Die chemische Modifikation kann z.B. in der Addition einer oder mehrerer bestimmter chemischer Gruppen an eine oder mehrere Aminosäuren bestehen.

Insulinanaloga, welche in EP 0 214 826, EP 0 375 437, EP 0 678 522, EP 0 885 961, EP 0 419 504, WO 92/00321, den deutschen Patentanmeldungen Nr. 10 2008 003 568.8 und Nr. 10 2008 003 566.1 und EP-A 0 368 187 beschrieben sind, können Bestandteil der hierin beschriebenen Zusammensetzungen sein.

Ein bevorzugtes hierin beschriebenes Insulinanalogon kann ausgewählt sein aus der Gruppe bestehend aus Gly(A21)-Arg(B31)-Arg(B32)-Humaninsulin (Insulin Glargin, Lantus); Arg(A0)-His(A8)-Glu(A15)-Asp(A18)-Gly(A21)-Arg(B31)-Arg(B32)-humaninsulinamid, Lys(B3)-Glu(B29)-Humaninsulin; Lys$^{B28}$Pro$^{B29}$Humaninsulin (Insulin Lyspro), B28 Asp-Humaninsulin (Insulin Aspart), Humaninsulin, bei dem Prolin in der Position B28 substituiert wurde durch Asp, Lys, Leu, Val oder Ala und wo in Position B29 Lys durch Pro substituiert sein kann; AlaB26-Humaninsulin; Des(B28-B30)-Humaninsulin; Des(B27)-Humaninsulin oder B29Lys($\epsilon$-tetradecanoyl),des(B30)-Humaninsulin (Insulin Detemir).

Ein bevorzugtes hierin beschriebenes Insulinderivat kann ausgewählt sein aus der Gruppe bestehend aus B29-N-myristoyl-des(B30) Humaninsulin, B29-N-palmitoyldes(B30) Humaninsulin, B29-N-myristoyl Humaninsulin, B29-N-palmitoyl Humaninsulin, B28-N-myristoyl Lys$^{B28}$Pro$^{B29}$ Humaninsulin, B28-N-palmitoyl-Lys$^{B28}$Pro$^{B29}$ Humaninsulin, B30-N-myristoyl-Thr$^{B29}$Lys$^{B30}$ Humaninsulin, B30-N-palmitoyl- Thr$^{B29}$Lys$^{B30}$ Humaninsulin, B29-N-(N-palmitoyl-Y-glutamyl)-des(B30) Humaninsulin, B29-N-(N-lithocholyl-Y-glutamyl)-des(B30) Humaninsulin, B29-N-($\omega$-carboxyheptadecanoyl)-des(B30) Humaninsulin und B29-N-($\omega$-carboxyheptadecanoyl) Humaninsulin.

Ein stärker bevorzugtes hierin beschriebenes Insulinderivat wird ausgewählt aus der Gruppe bestehend aus Gly(A21)-Arg(B31)-Arg(B32)-Humaninsulin, Lys$^{B28}$Pro$^{B29}$ Humaninsulin (Insulin Lyspro), B28 Asp Humaninsulin (Insulin Aspart), B29Lys($\varepsilon$-tetradecanoyl),desB30 Humaninsulin (Insulin Detemir).

Der Begriff "wenigstens ein Insulin" schließt Kombinationen der hierin beschriebenen Insuline, Analoga, Derivate und Metabolite davon ein, welche in den hierin beschriebenen Zusammensetzungen eingesetzt werden, z. B. beliebige Kombinationen von zwei oder mehr ausgewählt aus den hierin beschriebenen Insuline, Analoga, Derivate und Metabolite.

Die hierin beschriebenen Zusammensetzungen enthalten 60-6000 nmol/ml, bevorzugt 240-3000 nmol/ml eines Insulins wie hierin definiert. Eine Konzentration von 240-3000 nmol/ml entspricht je nach verwendetem Insulin etwa einer Konzentration 1,4-35 mg/ml oder 40-500 Einheiten/ml.

Im 2- bis 10-, bevorzugt 3- bis 5-, pens-cover-all Konzept liegen die Zusammensetzungen im Bereich von 20 $\mu$g/ml GLP-1 Agonist und 100 U/ml Insulin bis 300 $\mu$g/ml GLP-1 Agonist und 500 U/ml Insulin. Bevorzugt sind folgende Konzentrationsbereiche: 25 $\mu$g/ml und 100 U/ml, 33 $\mu$g/ml und 100 U/ml, 40 $\mu$g/ml und 100 U/ml, 66 $\mu$g/ml und 100 U/ml sowie 75 $\mu$g/ml und 100 U/ml.

Der gewünschte Dosierungsbereich des Insulins ist insbesondere eine Dosierung mit synergistischer Wirkung. Hier liegen die Werte bei 5 bis 100 U, bevorzugt bei 15 bis 80 U. Für den GLP-1 Agonisten liegen die Werte für den Dosierungsbereich bei 5 $\mu$g bis 2 mg, bevorzugt bei 10 $\mu$g bis 1,8 mg, besonders bevorzugt bei 10 $\mu$g bis 30 $\mu$g.

Die bevorzugte Darreichungsform der pharmazeutischen Zusammensetzungen der vorliegenden Erfindung sind flüssige Zusammensetzungen, welche insbesondere zur parenterale Verabreichung, besonders bevorzugt zur Injektion, am stärksten bevorzugt zur subkutanen Injektion geeignet sind. Insbesondere ist die pharmazeutische Zusammensetzung der vorliegenden Erfindung zur Injektion einmal täglich geeignet.

Die pharmazeutische Zusammensetzung der vorliegenden Erfindung kann einen sauren oder physiologischen pH aufweisen. Ein saurer pH Bereich liegt vorzugsweise im Bereich von pH 1 - 6,8, stärker bevorzugt pH 3,5 - 6,8, noch stärker bevorzugt pH 3,5 - 4,5, am stärksten bevorzugt bei einem pH von etwa 4,0-4,5. Ein physiologischer pH liegt bevorzugt im Bereich von pH 4,0 -8,5, stärker bevorzugt pH 5,0 bis 8,5, noch stärker bevorzugt pH 6,0 bis 8,5.

Die erfindungsgemäße Zusammensetzung kann ein geeignetes Konservierungsmittel enthalten. Geeignete Konservierungsmittel sind z.B. Phenol, m-Cresol, Benzylalkohol und/oder p-Hydroxybenzoesäureester.

Ferner kann die erfindungsgemäße Zusammensetzung einen geeigneten Puffer enthalten. Als Puffersubstanzen, insbesondere zur Einstellung eines pH-Wertes zwischen etwa 4,0 und 8,5 können z.B. Natriumacetat, Natriumcitrat, Natriumphosphat etc. verwendet werden. Ansonsten sind zur Einstellung des pH-Wertes auch physiologisch unbedenkliche verdünnte Säuren (typischerweise HCl) bzw. Laugen (typischerweise NaOH) geeignet. Bevorzugte Konzentrationen der Puffer sowie entsprechender Salze liegen im Bereich von 5 - 250 mM, stärker bevorzugt im Bereich von 10-100 mM. Die erfindungsgemäße Zusammensetzung kann Zinkionen enthalten. Die Konzentration der Zinkionen liegt vorzugsweise im Bereich von 0 $\mu$g/ml bis 500$\mu$g/ml, stärker bevorzugt von 5 $\mu$g bis 200 $\mu$g Zink/ml.

Ferner kann die erfindungsgemäße Zusammensetzung geeignete Isotonisierungsmittel enthalten. Geeignet sind z.B. Glycerol, Dextrose, Lactose, Sorbitol, Mannitol, Glukose, NaCl, Calcium- oder Magnesium-Verbindungen wie CaCl$_2$ etc. Glycerol, Dextrose, Lactose, Sorbitol, Mannitol und Glukose liegen üblicherweise im Bereich von 100 - 250 mM, NaCl in einer Konzentration von bis zu 150 mM.

Ferner kann die erfindungsgemäße Zusammensetzung ein Tensid enthalten. Ein Tensid kann die Stabilität von sauren Insulinzusammensetzungen stark erhöhen. Damit können sogar Zusammensetzungen hergestellt werden, die über mehrere Monate bei Temperaturbelastung die überlegene Stabilität gegenüber hydrophoben Aggregationskeimen garantieren.

Das Tensid wird vorzugsweise ausgewählt aus der Gruppe bestehend aus Partial- und Fettsäureester und -ether mehrwertiger Alkohole wie des Glycerols und Sorbitols, Polyole; wobei die Partial- und Fettsäureester und -ether des Glycerols und Sorbitols ausgewählt werden aus einer Gruppe enthaltend Span®, Tween®, Myrj®, Brij®, Cremophor®; wobei die Polyole ausgewählt werden aus der Gruppe Polypropylenglycole, Polyethylenglycole, Poloxamere, Polysorbate, Pluronics, Tetronics. Bevorzugte Konzentrationen der Tenside liegen im Bereich von 5 - 200 $\mu$g/ml, bevorzugt von 5 -120$\mu$g/ml und besonders bevorzugt von 20 - 75 $\mu$g/ml vor.

Ferner kann die erfindungsgemäße Zusammensetzung weitere Zusätze wie z.B. Salze enthalten, welche die Freigabe des wenigstens einen Insulins retardieren.

Ein hierin beschriebener Gegenstand ist ein Arzneimittel wie hierin beschrieben umfassend wenigstens ein Insulin unabhängig ausgewählt aus Lys$^{B28}$Pro$^{B29}$ Humaninsulin (Insulin Lyspro), B28 Asp Humaninsulin (Insulin Aspart), B29Lys($\varepsilon$-tetradecanoyl),desB30 Humaninsulin (Insulin Detemir), und Insulin Glargin (Gly(A21)-Arg(B31)-Arg(B32)-Humaninsulin), und umfassend AVE0010 oder/und ein pharmakologisch tolerierbares Salz davon. Ein weiterer besonders bevorzugter Gegenstand ist ein Arzneimittel wie hierin beschrieben umfassend Insulin Glargin (Gly(A21)-Arg(B31)-Arg(B32)-Humaninsulin) und AVE0010 (des Pro$^{36}$Exendin-4(1-39)-Lys$_6$-NH$_2$) oder/und ein pharmakologisch tolerierbares Salz davon. Die Zusammensetzungen dieser besonders bevorzugten Arzneimittel weisen vorzugsweise einen sauren pH von 1 - 6,8, stärker bevorzugt pH 3,5 - 6,8, noch stärker bevorzugt pH 3,5 - 5,0, am stärksten bevorzugt bei einem pH von etwa 4,0 bis 4,5. Ferner können die Zusammensetzungen dieser besonders bevorzugten Arzneimittel ein Tensid wie hierin beschrieben enthalten.

Ein weiterer erfindungsgemäßer Gegenstand ist eine Kombination aus Insulin Glargin (Gly(A21)-Arg(B31)-Arg(B32)-Humaninsulin) und AVE0010 (des Pro$^{36}$Exendin-4(1-39)-Lys$_6$-NH$_2$) oder/und einem pharmakologisch tolerierbaren Salz davon.

Insbesondere ist ein Gegenstand der Erfindung eine pharmazeutische Kombination umfassend Gly(A21)-Arg(B31)-Arg(B32)-Humaninsulin oder/und ein pharmakologisch tolerierbares Salz davon in einer Konzentration von 100 bis 500 U/ml.

Ein weiterer Gegenstand der Erfindung ist eine pharmazeutische Kombination umfassend desPro$^{36}$Exendin-4(1-39)-Lys$_6$-NH$_2$ oder/und ein pharmakologisch tolerierbares Salz davon in einer Konzentration von 20 bis 150 $\mu$g/ml.

Noch ein weiterer Gegenstand der Erfindung ist eine pharmazeutische Zusammensetzung, umfassend Gly(A21)-Arg(B31)-Arg(B32)-Humaninsulin oder/und ein pharmakologisch tolerierbares Salz davon in einer Konzentration von 100 bis 500 U/ml.

Ein weiterer Gegenstand der Erfindung ist eine pharmazeutische Zusammensetzung, umfassend desPro$^{36}$Exendin-4(1-39)-Lys$_6$-NH$_2$ oder/und ein pharmakologisch tolerierbares Salz davon in einer Konzentration von 20 bis 150 $\mu$g/ml.

Ein weiterer hierin beschriebener Gegenstand ist Verfahren zur Behandlung eines Patienten mit einem erfindungsgemäßen Arzneimittel oder Kit wie hierin beschrieben.

Das hierin beschriebene Verfahren zur Behandlung eines Patienten umfasst die Verabreichung eines hierin beschriebenen Arzneimittels umfassend wenigstens ein Insulin und wenigstens einen GLP-1-Agonisten, wobei das Arzneimittel so formuliert oder/und konfektioniert ist, dass es das Insulin und den GLP-1-Agonisten in einer jeweils vorbestimmten Menge enthält und in einer jeweils an den Bedarf eines Patienten angepassten Dosis verabreicht werden kann.

Insbesondere umfasst das Verfahren die Verabreichung eines Arzneimittels umfassend eine erste pharmazeutische Zusammensetzung und eine zweite pharmazeutische Zusammensetzung und gegebenenfalls wenigstens eine weitere pharmazeutische Zusammensetzung, welche jeweils wenigstens ein Insulin und wenigstens einen GLP-1-Agonisten umfassen und das wenigstens eine Insulin oder/und den wenigstens einen GLP-1-Agonisten in unterschiedlichen Gewichtsanteilen bezogen auf das Gesamtgewicht der Zusammensetzung enthalten, wobei das Verfahren umfasst:

(a) Auswählen einer Dosis des wenigstens einen Insulins, welche verabreicht werden soll,
(b) Auswählen einer Dosis des wenigstens einen GLP-1-Agonisten, welche verabreicht werden soll,
(c) Auswählen einer Zusammensetzung aus der ersten, zweiten und gegebenenfalls wenigstens einen weiteren Zusammensetzung des Arzneimittels, welche die Dosen aus (a) und (b) in einer Konzentration enthält, so dass die Dosen aus (a) und (b) in gleichem Volumen vorliegen, und
(d) Bestimmen und Verabreichen einer Menge, welche den Dosen aus (a) und (b) entspricht.

Die Bestimmung der Dosis nach Schritt (a) oder/und Schritt (b) erfolgt nach dem individuellen Bedarf der Patienten.

Schritt (c) des hierin beschriebenen Behandlungsverfahrens kann anhand einer Tabelle durchgeführt werden. Diese Tabelle kann Bestandteil des hierin beschriebenen Arzneimittels sein. Beispiel 11 enthält ein Beispiel für eine hierin beschriebene Tabelle.

Das Verfahren zur Behandlung eines Patienten kann insbesondere die Verabreichung eines Arzneimittels umfassen, wobei das Arzneimittel eine erste pharmazeutische Zusammensetzung und eine zweite pharmazeutische Zusammensetzung und gegebenenfalls wenigstens eine weitere pharmazeutische Zusammensetzung umfasst, wobei die erste pharmazeutische Zusammensetzung wenigstens einen ersten Wirkstoff umfasst, und wobei die zweite pharmazeutische Zusammensetzung wenigstens einen ersten Wirkstoff und wenigstens einen zweiten Wirkstoff umfasst, und wobei die wenigstens eine weitere pharmazeutische Zusammensetzung wenigstens einen ersten Wirkstoff und wenigstens einen weiteren Wirkstoff umfasst, und wobei das Verfahren die Schritte umfasst:

(i) Auswählen einer Dosis des wenigstens einen ersten Wirkstoffs, welche verabreicht werden soll, und Bestimmen der Gesamtmenge der ersten, zweiten und gegebenenfalls wenigstens einen weiteren Zusammensetzung, so dass die ausgewählte Dosis des wenigstens einen ersten Wirkstoffs in der Gesamtmenge enthalten ist,
(ii) Auswählen einer Dosis des wenigstens einen zweiten Wirkstoffs, welche verabreicht werden soll und Bestimmen der Menge der zweiten Zusammensetzung, so dass die ausgewählte Dosis des wenigstens einen zweiten Wirkstoffs in der Menge der zweiten Zusammensetzung enthalten ist,
(iii) gegebenenfalls Auswählen einer Dosis des wenigstens einen weiteren Wirkstoffs, welche verabreicht werden soll, und Bestimmen der Menge der wenigstens einen weiteren Zusammensetzung, so dass die ausgewählte Dosis des wenigstens einen weiteren Wirkstoffs in der Menge der wenigstens einen weiteren Zusammensetzung enthalten ist,

(iv) Verabreichen einer Menge der ersten Zusammensetzung an den Patienten, wobei die verabreichte Menge der Gesamtmenge gemäß Schritt (i) abzüglich der Menge der zweiten Zusammensetzung gemäß Schritt (ii) und gegebenenfalls abzüglich der Menge der wenigstens einen weiteren Zusammensetzung gemäß Schritt (iii) entspricht, und

(v) Verabreichen der Menge der zweiten Zusammensetzung, welche in Schritt (ii) bestimmt wurde, und gegebenenfalls der Menge der wenigstens einen weiteren Zusammensetzung, welche in Schritt (iii) bestimmt wurde, an den Patienten.

Der erste Wirkstoff kann ein Insulin sein, und der zweite Wirkstoff kann ein GLP-1-Agonist sein. Damit kann das Verfahren zur Behandlung eines Patienten insbesondere die Verabreichung eines Arzneimittels umfassen, wobei das Arzeimittel eine erste pharmazeutische Zusammensetzung und eine zweite pharmazeutische Zusammensetzung und gegebenenfalls wenigstens eine weitere pharmazeutische Zusammensetzung umfasst, wobei die erste pharmazeutische Zusammensetzung wenigstens ein Insulin umfasst, und wobei die zweite pharmazeutische Zusammensetzung wenigstens ein Insulin und wenigstens einen GLP-1-Agonisten umfasst, und wobei die wenigstens eine weitere pharmazeutische Zusammensetzung wenigstens ein Insulin und wenigstens einen weiteren Wirkstoff umfasst, und wobei das Verfahren die Schritte umfasst:

(i) Auswählen einer Dosis des wenigstens einen Insulins, welche verabreicht werden soll, und Bestimmen der Gesamtmenge der ersten, zweiten und gegebenenfalls wenigstens einen weiteren Zusammensetzung, so dass die ausgewählte Dosis des wenigstens einen Insulins in der Gesamtmenge enthalten ist,

(ii) Auswählen einer Dosis des wenigstens einen GLP-1-Agonisten, welche verabreicht werden soll und Bestimmen der Menge der zweiten Zusammensetzung, so dass die ausgewählte Dosis des wenigstens einen GLP-1-Agonisten in der Menge der zweiten Zusammensetzung enthalten ist,

(iii) gegebenenfalls Auswählen einer Dosis des wenigstens einen weiteren Wirkstoffs, welche verabreicht werden soll, und Bestimmen der Menge der wenigstens einen weiteren Zusammensetzung, so dass die ausgewählte Dosis des wenigstens einen weiteren Wirkstoffs in der Menge der wenigstens einen weiteren Zusammensetzung enthalten ist,

(iv) Verabreichen einer Menge der ersten Zusammensetzung an den Patienten, wobei die verabreichte Menge der Gesamtmenge gemäß Schritt (i) abzüglich der Menge der zweiten Zusammensetzung gemäß Schritt (ii) und gegebenenfalls abzüglich der Menge der wenigstens einen weiteren Zusammensetzung gemäß Schritt (iii) entspricht, und

(v) Verabreichen der Menge der zweiten Zusammensetzung, welche in Schritt (ii) bestimmt wurde, und gegebenenfalls der Menge der wenigstens einen weiteren Zusammensetzung, welche in Schritt (iii) bestimmt wurde, an den Patienten.

Der erste Wirkstoff kann ein GLP-1-Agonist sein, und der zweite Wirkstoff kann ein Insulin sein. Damit kann das Verfahren zur Behandlung eines Patienten insbesondere die Verabreichung eines Arzneimittels umfassen, wobei das Arzeimittel eine erste pharmazeutische Zusammensetzung und eine zweite pharmazeutische Zusammensetzung und gegebenenfalls wenigstens eine weitere pharmazeutische Zusammensetzung umfasst, wobei die erste pharmazeutische Zusammensetzung wenigstens einen GLP-1-Agonisten umfasst, und wobei die zweite pharmazeutische Zusammensetzung wenigstens einen GLP-1-Agonisten und wenigstens ein Insulin umfasst, und wobei die wenigstens eine weitere pharmazeutische Zusammensetzung wenigstens einen GLP-1-Agonisten und wenigstens einen weiteren Wirkstoff umfasst, und wobei das Verfahren die Schritte umfasst:

(i) Auswählen einer Dosis des wenigstens einen GLP-1-Agonisten, welche verabreicht werden soll, und Bestimmen der Gesamtmenge der ersten, zweiten und gegebenenfalls wenigstens einen weiteren Zusammensetzung, so dass die ausgewählte Dosis des wenigstens einen GLP-1-Agonisten in der Gesamtmenge enthalten ist,

(ii) Auswählen einer Dosis des wenigstens einen Insulins, welche verabreicht werden soll und Bestimmen der Menge der zweiten Zusammensetzung, so dass die ausgewählte Dosis des wenigstens einen Insulins in der Menge der zweiten Zusammensetzung enthalten ist,

(iii) gegebenenfalls Auswählen einer Dosis des wenigstens einen weiteren Wirkstoffs, welche verabreicht werden soll, und Bestimmen der Menge der wenigstens einen weiteren Zusammensetzung, so dass die ausgewählte Dosis des wenigstens einen weiteren Wirkstoffs in der Menge der wenigstens einen weiteren Zusammensetzung enthalten ist,

(iv) Verabreichen einer Menge der ersten Zusammensetzung an den Patienten, wobei die verabreichte Menge der Gesamtmenge gemäß Schritt (i) abzüglich der Menge der zweiten Zusammensetzung gemäß Schritt (ii) und gegebenenfalls abzüglich der Menge der wenigstens einen weiteren Zusammensetzung gemäß Schritt (iii) entspricht, und

(v) Verabreichen der Menge der zweiten Zusammensetzung, welche in Schritt (ii) bestimmt wurde, und gegebenenfalls der Menge der wenigstens einen weiteren Zusammensetzung, welche in Schritt (iii) bestimmt wurde, an den Patienten.

Die Schritte (i), (ii) oder/und (iii) können anhand von mindestens einer Tabelle durchgeführt werden, welche Bestandteil des Arzneimittels sein kann. Für jeden der Schritte (i), (ii) und (iii) kann unabhängig voneinander eine Tabelle vorgesehen sein.

Das hierin beschriebene Behandlungsverfahren kann insbesondere zur Behandlung von Patienten mit Diabetes, insbesondere mit Diabetes Typ 1 oder II eingesetzt werden. Bevorzugt wird das Verfahren zur Einstellung der nüchternen, der postprandialen oder/und der postabsorptiven Blutglukosekonzentration, zur Verbesserung der Glukosetoleranz, zur Prävention einer Hypoglykämie, zur Prävention eines Funktionsverlustes der $\beta$-Zellen des Pankreas, zur Gewichtsabnahme oder/und zur Prävention einer Gewichtszunahme eingesetzt.

Hierin beschrieben ist ein Verfahren zur Herstellung eines hierin beschriebenen Arzneimittels umfassend Formulieren oder/und Konfektionieren, so dass es das Insulin und den GLP-1-Agonisten in einer jeweils vorbestimmten Menge enthält und in einer jeweils an den Bedarf eines Patienten angepassten Dosis verabreicht werden kann. Bevorzugt wird im Herstellungsverfahren das Arzneimittel so formuliert und konfektioniert, dass eines der hierin beschriebenen Arzneimittel erhalten werden kann, zum Beispiel ein Arzneimittel umfassend eine erste pharmazeutische Zusammensetzung und eine zweite pharmazeutische Zusammensetzung und gegebenenfalls wenigstens eine weitere pharmazeutische Zusammensetzung, welche jeweils wenigstens ein Insulin und wenigstens einen GLP-1-Agonisten umfassen und das wenigstens eine Insulin oder/und den wenigstens einen GLP-1-Agonisten in unterschiedlichen Gewichtsanteilen bezogen auf das Gesamtgewicht der Zusammensetzung enthalten.

Ein weiterer erfindungsgemäßer Gegenstand ist ein Verfahren zur Herstellung eines erfindungsgemäßen Zusammensetzung, umfassend Formulieren oder/und Konfektionieren von Gly(A21)-Arg(B31)-Arg(B32)-Humaninsulin und desPro$^{36}$Exendin-4(1-39)-Lys$_6$-NH$_2$.

Die Erfindung wird durch die folgenden Abbildungen und das folgende Beispiel illustriert, ohne dass diese die Erfindung in irgend einer Weise beschränken.

Legenden zu den Abbildungen

Fig. 1: Studiendesign für den oralen Glukose-Toleranztest.

Fig. 2: OGTT beim Hund: Wirkung von Insulin Glargin gegenüber Placebo.

Fig. 3: OGTT beim Hund: Wirkung von AVE0010 gegenüber Placebo.

Fig. 4: OGTT beim Hund: Wirkung einer AVE0010/Insulin Glargin-Kombination auf den Blutglukosespiegel.

Fig. 5: OGTT beim Hund: Wirkung einer AVE0010-Insulin Glargin-Kombination auf das Plasmainsulin und den c-Peptidspiegel.

Fig. 6: OGTT beim Hund: Wirkung einer Dosisabsenkung von AVE0010 mit verschiedenen Verhältnissen zu Insulin Glargin in der Kombinationsformulierung.

Fig. 7: Wirkung einer AVE0010-Insulin Glargin-Kombination auf die Blutglukose in der diabetischen db/db-Maus.

Fig.8: Wirkung einer AVE0010-Insulin Glargin-Kombination im oralen Glukose-Toleranztest in der diabetischen db/db-Maus.

Fig. 9: Wirkung der AVE0010-Insulin Glargin-Kombination auf die Cytokin- und Lipotoxizitäts-induzierte $\beta$-Zell-Apoptose in vitro.

Fig. 10: Das "3 pens cover all"-Konzept.

Beispiele

Beispiel 1

Modell: oraler Glukose-Toleranztest (OGTT) in gesunden Hunden: Vergleich der Kombination Insulin Glargin-AVE0010 mit den beiden einzelnen Wirkstoffen.

Tiere

- Männliche normoglykämische Beagles
- Körpergewicht: ~15 kg
- Anzahl pro Gruppe: n = 6

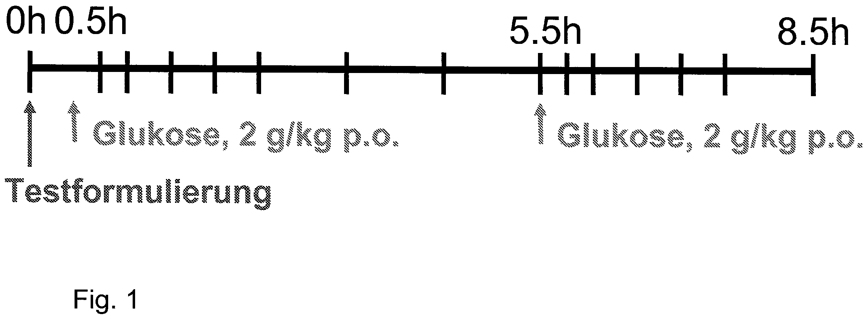

Studiendesign (siehe Fig. 1)

- Einzelne subkutane Injektionen von Placebo- oder Testformulierung zum Zeitpunkt 0
- 2 orale Glukosegaben von 2 g Glukose/kg Körpergewicht zum Zeitpunkt 30 min und 5h
- Blutproben werden zur Bestimmung von Blutglukose, Plasmainsulin und c-Peptid genommen

Gruppeneinteilung (n = 6)

- Placebo (= Lantus-Placeboformulierung ohne API)
- Insulin Glargin (0.3 IU/kg s.c., äquivalent zu 1,8 nmol/kg). Insulin Glargin ist Gly(A21)-Arg(B31)-Arg(B32)-Huma-ninsulin.
- AVE0010 (10 $\mu$g/kg s.c. in Lantus-Placeboformulierung, äquivalent zu 2 nmol/kg). AVE0010 ist des Pro[36] Exendin-4(1-39)-Lys$_6$-NH$_2$.
- AVE0010-Insulin Glargin-Kombination (10 $\mu$g/kg AVE0010 / 0.3 IU/kg Insulin Glargin s.c.)

Beispiel 2

OGTT beim Hund: Wirkung von Insulin Glargin gegenüber Placebo
Der Versuch wurde nach dem in Beispiel 1 beschriebenen Protokoll durchgeführt.

- wiederholter OGTT (2g/kg p.o.)
- männl. Beagle, n = 6
- MW $\pm$ Sem
- Placebo = Lantus-Placebo
- Insulin Glargin (0.3 U/kg s.c.)

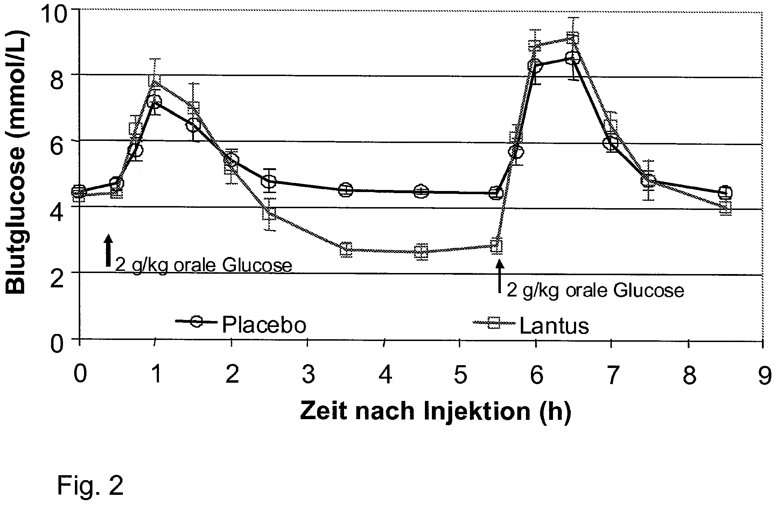

Ergebnis: Die Daten sind in Fig. 2 dargestellt. Die alleinige Gabe von Insulin Glargin verhindert den OGTT-induzierten Anstieg der Blutglukose nicht. Insulin Glargin bewirkt eine Verstärkung der erwarteten verzögerten Senkung der Blutglukosekonzentration in der postabsorptiven Phase.

Beispiel 3

OGTT beim Hund: Wirkung von AVE0010 gegenüber Placebo
Der Versuch wurde nach dem in Beispiel 1 beschriebenen Protokoll durchgeführt.

- wiederholter OGTT (2g/kg p.o.)
- männl. Beagle, n = 6
- MW $\pm$ Sem
- Placebo = Lantus-Placebo

- AVE0010 (10 μg/kg s.c.)

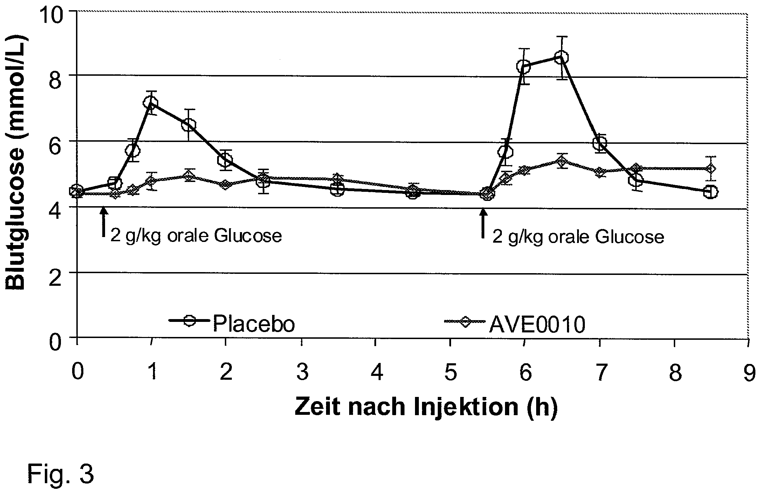

Ergebnis: Die Daten sind in Fig. 3 dargestellt. AVE0010 verhindert den OGTT-induzierten postprandialen Anstieg der Blutglukose fast vollständig. Eine Wirkung auf die Glukosekonzentration in der postabsorptiven Phase fehlt. Dieses Beispiel zeigt, dass die Wirkung von AVE0010 auf den OGTT-induzierten postprandialen Anstieg der Blutglukose komplementär zur blutzuckersenkenden Wirkung von Insulin Glargin in der postabsorptiven Phase ist.

Beispiel 4

OGTT beim Hund: Wirkung einer AVE0010-Insulin Glargin-Kombination auf den Blutglukosespiegel
Der Versuch wurde nach dem in Beispiel 1 beschriebenen Protokoll durchgeführt.

- wiederholter OGTT (2g/kg p.o.)
- männl. Beagle, n = 6
- MW ± Sem
- Placebo = Lantus-Placebo
- AVE0010 (10 μg/kg s.c.)
- Insulin Glargin (0.3 U/kg s.c.)
- AVE+Lan (= Prämix von 10 μg/kg AVE0010 und 0.3 U/kg Insulin Glargin in einer Formulierung)

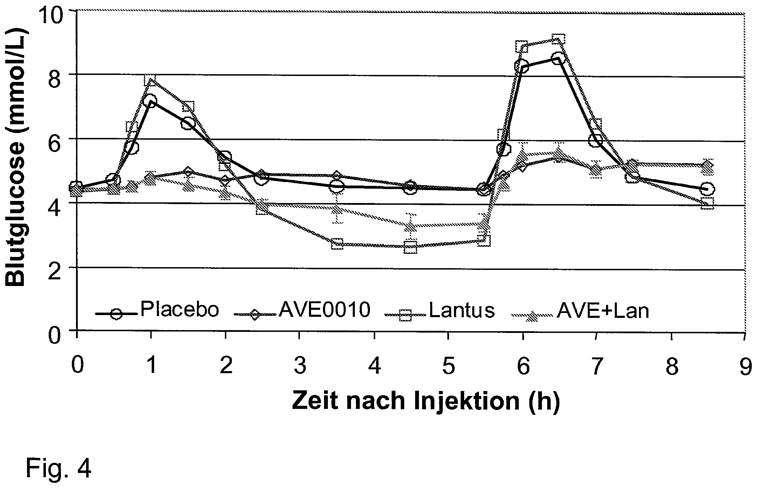

Ergebnis: Die Daten sind in Fig. 4 dargestellt. Die Kombination wirkt auf den postprandialen Glukoseanstieg wie AVE0010 (vergl. Beispiel 3). Der hypoglykämischer Effekt von Insulin Glargin in der postabsorptiven Phase ist ebenfalls vorhanden, aber abgeschwächt (vergl. Beispiel 2). Dies ist ein synergistischer Effekt von Insulin Glargin und AVE0010, da AVE0010 allein keine Wirkung auf den nach Glukosegabe wieder abgesunkenen Glukosespiegel hat, und Insulin Glargin alleine keine Wirkung auf den postprandialen Glukosespiegel hat.

Beispiel 5

OGTT beim Hund: Wirkung einer AVE0010-Insulin Glargin-Kombination auf das Plasmainsulin und den c-Peptidspiegel
Der Versuch wurde nach dem in Beispiel 1 beschriebenen Protokoll durchgeführt.

- wiederholter OGTT (2g/kg p.o.)
- männl. Beagle, n = 6
- MW ± Sem
- Placebo = Lantus-Placebo
- AVE0010 (10 μg/kg s.c.)
- Insulin Glargin (0.3 U/kg s.c.)
- AVE+Lan (= Prämix von 10 μg/kg AVE0010 und 0.3 U/kg Insulin Glargin in einer Formulierung)

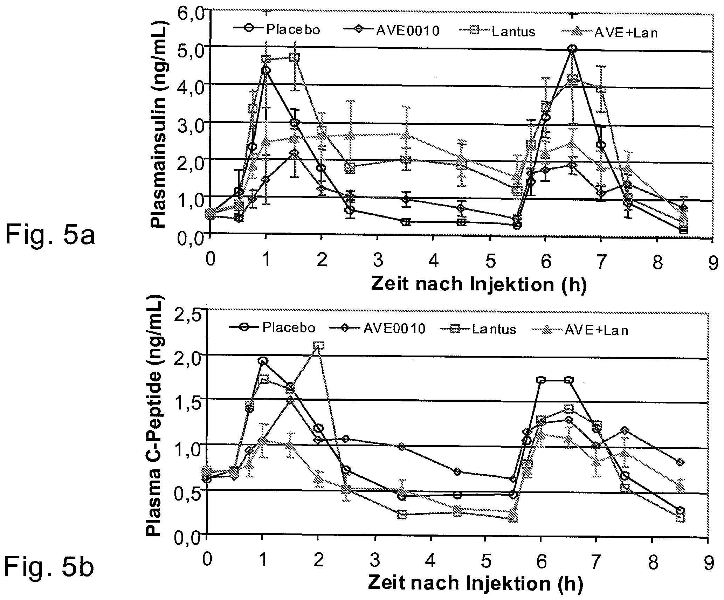

Das C-Peptid wird bei der Umwandlung von Proinsulin in Insulin freigesetzt und dient als Marker der Insulinsekretion der β-Zellen des Pankreas. Im Rahmen eines Glukosebelastungstests kann mithilfe des c-Peptids die Reaktionsfähigkeit des Pankreas bestimmt werden.
Ergebnis: Die Daten sind in Fig. 5a und 5b dargestellt. In der Kombinationsgruppe folgt auf die postprandiale Insulinreduktion ein erhöhter postabsorptiver Insulin Glarginspiegel. C-Peptidspiegel der Kombination entsprechen der Insulinkurve von AVE0010 während der prandialen Phasen und von Insulin Glargin während der postabsorptiven Phase.

Beispiel 6

OGTT beim Hund: Wirkung einer Dosisabsenkung von AVE0010 mit verschiedenen Verhältnissen zu Insulin Glargin in der Kombinationsformulierung.
Der Versuch wurde nach dem in Beispiel 1 beschriebenen Protokoll durchgeführt.

- wiederholter OGTT (2g/kg p.o.)
- männl. Beagle, n =11/6/6/6
- MW ± Sem
- Kontrolle = Lantus-Placebo
- AVE+Lan (= Prämix von 0.15 bis 1.0 μg/kg AVE0010 und 0.3 U/kg Insulin Glargin in einer Formulierung). In den Beispielen 2 bis 5 wurden AVE0010-Konzentrationen von 10 µg/kg verwendet.

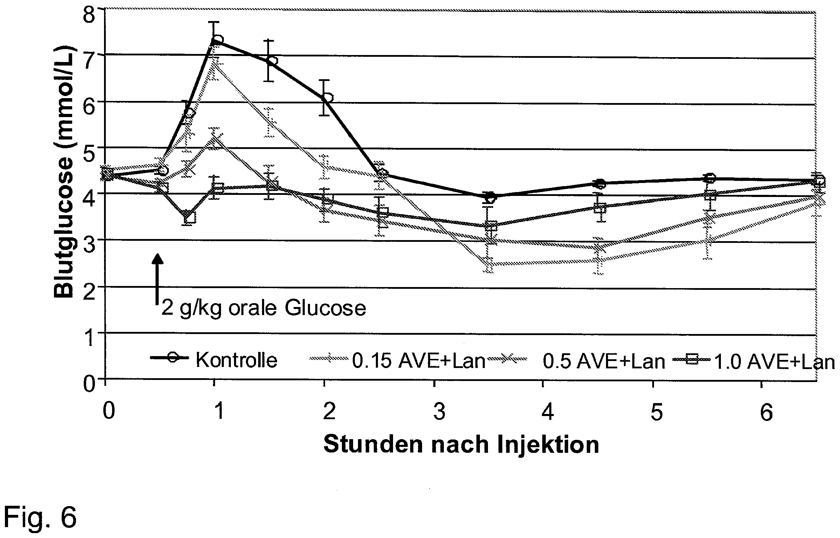

Ergebnis: Die Daten sind in Fig. 6 dargestellt. Eine Reduktion der AVE0010-Dosis von 10 µg/kg (vergl. insbesondere Beispiel 4) auf 1 µg/kg (d.h. um den Faktor 10) und die sich daraus ergebene Vergrößerung des Verhältnisses von Insulin Glargin zu AVE0010 wirkt sich nicht auf die synergistische Aktivität der Kombination AVE0010 mit Insulin Glargin aus (vergl. insbesondere Beispiel 4). Erst bei deutlich kleineren AVE0010-Dosen nähert sich die Wirkung der Kombination der Wirkung von Insulin Glargin allein an (vgl. insbesondere mit Fig. 2). Die AVE0010-Dosis kann also mindestens innerhalb einer Größenordnung (d.h. mindestens um den Faktor 10) variiert werden, ohne dass die synergistische Wirkung verlorengeht.

Beispiel 7

Modell: Diabetische insulin-resistente db/db-Maus: Vergleich der Kombination Insulin Glargin-AVE0010 mit den beiden einzelnen Wirkstoffen.

Tiere

- Weibliche db/db-Maus

- Alter: 10-11 Wochen

- Anzahl pro Gruppe: n = 10

Studiendesign

- Einzelne subkutane Injektion von Placebo oder der Testformulierung
- Entnahme von Blutproben zur Bestimmung der Blutglukose

Gruppeneinteilung

- Placebo (= Lantus-Placeboformulierung ohne API)
- AVE0010 (10 µg/kg s.c.)
- Insulin Glargin (5 IU/kg s.c.)
- AVE0010-Insulin Glargin-Kombination (Prämix aus 10 µg/kg AVE0010 plus 5 IU/kg Insulin Glargin s.c.)

Beispiel 8

Wirkung einer AVE0010-Insulin Glargin-Kombination auf die Blutglukose in der diabetischen db/db-Maus
Der Versuch wurde nach dem in Beispiel 7 beschriebenen Protokoll durchgeführt.

- Weibliche db/db-Maus, 10 Wochen
- n = 10, MW ± Sem
- Vehikel = Lantus-Placebo
- AVE0010 (10 µg/kg sc)
- Lantus (5 U/kg sc)
- AVE0010-Insulin Glargin (= Prämix von AVE0010 10 µg/kg und Insulin Glargin 5 U/kg in einer Formulierung)

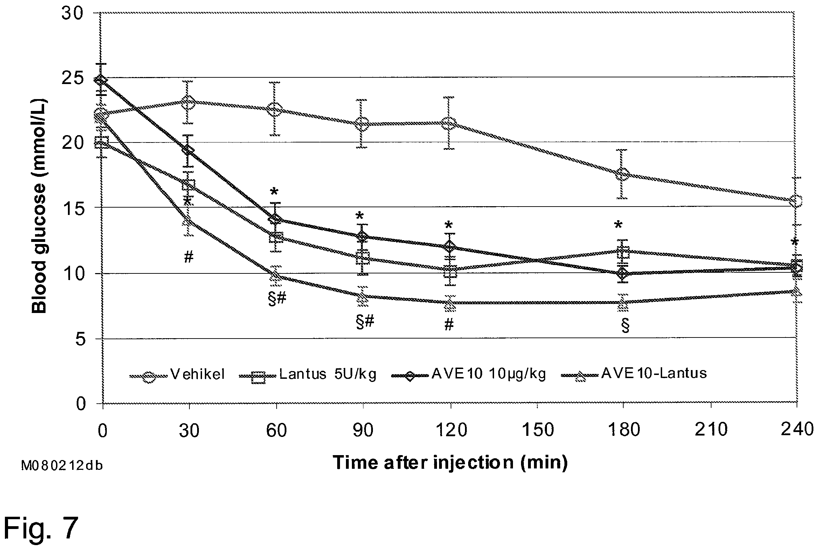

Ergebnis: Die Daten sind in Fig. 7 dargestellt. Die Kombination AVE0010-Insulin Glargin bewirkte in diabetischen db/db-Mäusen ein im Vergleich zu den beiden Einzelwirkstoffen eine schnellere und stärkere Abnahme der Blutglukosekonzentration. Damit führt die Kombination diabetische db/db-Mäuse dichter an die Normoglykämie heran als jeder der beiden Wirkstoffe allein.

Beispiel 9

Wirkung einer AVE0010-Insulin Glargin-Kombination im oralen Glukose-Toleranztest in der diabetischen db/db-Maus

Der Versuch wurde nach dem in Beispiel 7 beschriebenen Protokoll durchgeführt. Zusätzlich wurde ein OGTT (2 g/kg p.o. @ 30 min) durchgeführt.

- Weibliche db/db-Maus, 11 Wochen
- n = 10, MW $\pm$ Sem
- Kontrolle = Lantus-Placebo
- AVE0010 (10 $\mu$g/kg sc)
- Insulin Glargin (5 U/kg sc)
- AVE0010-Insulin Glargin (= Prämix von AVE0010 10 $\mu$g/kg und Insulin Glargin 5 U/kg in einer Formulierung)

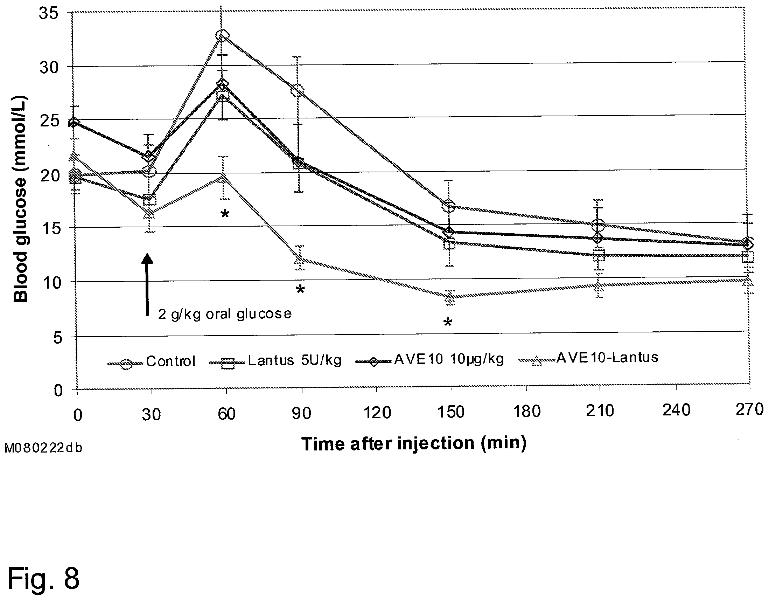

Ergebnis: Die Daten sind in Fig. 8 dargestellt. Die AVE0010-Insulin Glargin-Kombination führt zu einer signifikant verbesserten Glukosetoleranz und niedrigeren postabsorptiven Glukosespiegeln.

Beispiel 10

Wirkung der AVE0010-Insulin Glargin-Kombination auf die Cytokin- und Lipotoxizitätsinduzierte $\beta$-Zell-Apoptose *in vitro*

- Insulinoma-Zelllinie INS-1 der Ratte
- Inkubation mit der Testverbindung für 5h
- Weitere Inkubation mit einem Cytokinmix für 22h(1 ng/mL IFN-$\gamma$ + 4 ng/mL IL-1$\beta$) oder
- Weitere Inkubation mit 0.5 mM FFA für 18h (Palmitate:BSA 3:1)

Als Maß für die Apoptose werden die Caspase-3-Aktivität und die Fragmentierung der Zellkerne verwendet, welche mit der Apotose korrelieren.

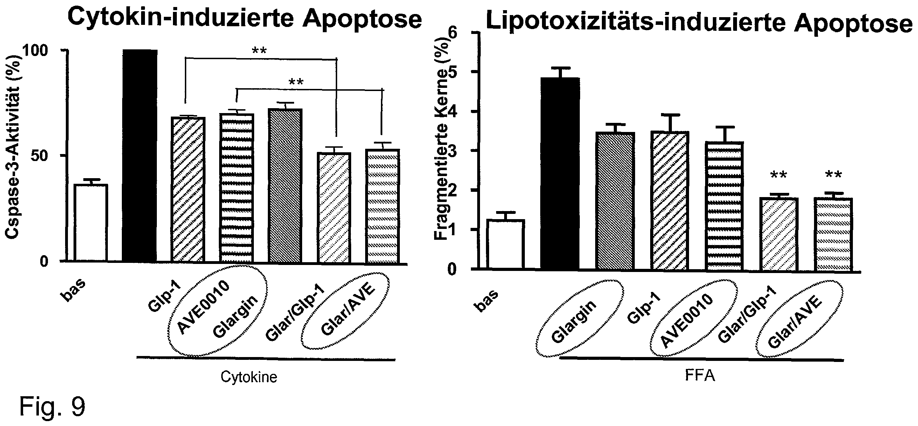

Ergebnis: Die Daten sind in Fig. 9 dargestellt. AVE0010 oder Insulin Glargin (=Glargin, Glar) allein verhindern die Apoptose um ~40-50%. Die Kombination AVE0010 und Insulin Glargin verhindern die Apotose signifikant besser. Die Kombination bewirkt aufgrund dieses synergistischen Effekts einen erhöhten Schutz gegen Cytokin- und Lipotoxizitäts-induzierter Apoptose.

Beispiel 11

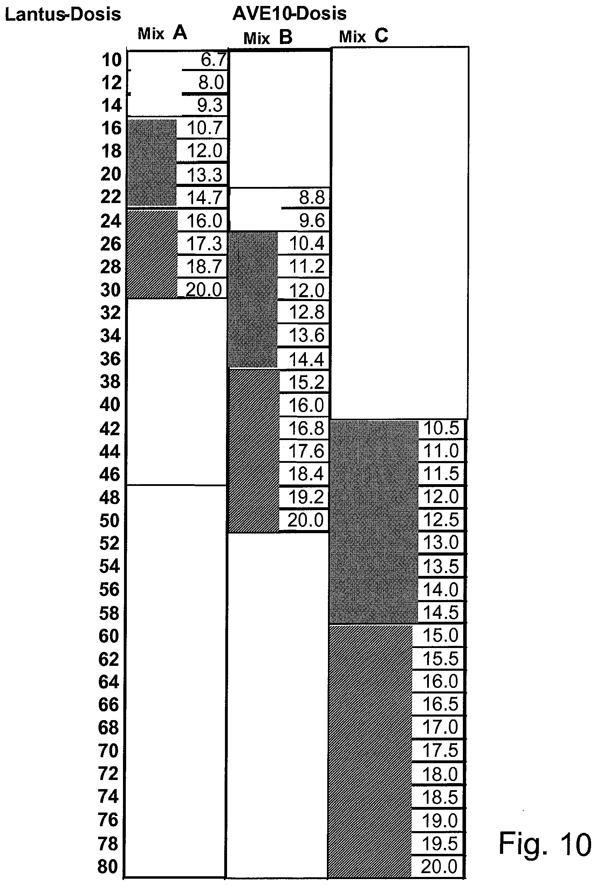

Das "3 pens cover all"-Konzept (Fig. 10)

- 3 Prämix-Stifte (Prämix-Pens) mit 3 verschiedenen vorbestimmten Verhältnissen:

  - Mix A: 100 U Insulin Glargin + 66.66 $\mu$g AVE0010 pro mL
  - Mix B: 100 U Insulin Glargin + 40 $\mu$g AVE0010 pro mL
  - Mix C: 100 U Insulin Glargin + 25 $\mu$g AVE0010 pro mL

- Einsatz der 3 Prämix-Stifte: Die beispielhafte Tabelle in Fig. 10 geht von einem therapeutischen Bereich von 15 bis 80 U pro Dosis Insulin Glargin und 10 bis 20 $\mu$g AVE0010 aus. Für einen bestimmten Patienten wird eine zu verabreichende Dosis von Insulin Glargin festgelegt bzw. vorbestimmt. Die vorbestimmte Dosis wird in der linken Spalte aufgesucht. Sofern in den Spalten MIX A - MIX C eine korrespondierende AVE0010-Dosis im Bereich zwischen 10 und 20 $\mu$g genannt ist, wird der entsprechende MIX ausgewählt, dosiert und verabreicht. Die Bereiche sind überlappend: z.B. könnte bei einem Bedarf von 26 bis 30 U Insulin Glargin Mix A oder MIX B (mit einer höheren Dosis AVE0010) gewählt werden. Entsprechendes gilt für MIX B und C. Wird zum Beispiel eine Dosis von 50 U Insulin bestimmt, so sind 0,5 ml von MIX B oder MIX C zu dosieren. Diese Dosis enthält 20 ug (MIX B) bzw. 12,5 $\mu$g (MIX C) AVE0010.

- Schlußfolgerung: Unter der Annahme, dass eine wahrscheinliche AVE0010-Wirkung zwischen 10 und 15 $\mu$g und eine therapeutische Wirkung zwischen 15 und 22 $\mu$g erzielt wird, können fast alle Patienten, welche Insulin Glargin-Dosen von 15-80 U nehmen, ebenso therapeutische Dosen von AVE0010 zwischen 10 und 20 $\mu$g erhalten, wenn sie einen der drei Prämixstifte verwenden, welche drei verschiedene Insulin Glargin:AVE0010 -Verhältnisse enthalten (Mix A, B or C). Aufgrund des breiten Bereichs möglicher Verhältnisse von Insulin Glargin zu AVE0010 (vergl. Beispiel 6) mit synergistischer Wirkung können die Verhältnisse in den Stiften so abgestimmt werden, dass für jede Dosis von Insulin Glargin in mindestens einem Stift eine synergistische Dosis AVE0010 enthalten ist.

Beispiel 12

Dieses Beispiel zeigt, auf welche Weise eine Kombination von zwei oder mehr Wirkstoffen so formuliert werden kann, dass bei Kombination von zwei oder mehr Zusammensetzungen beide Wirkstoffe in beliebigen Mengen und in beliebigen Verhältnissen zueinander verabreicht werden können. Hierbei wird berücksichtigt, dass mindestens einer der Wirkstoffe durch die Kombination (z.B. durch Mischen direkt vor der Verabreichung) nicht verdünnt werden darf.

Hierbei stehen die Bezeichnungen "Wirkstoff A" und "Wirkstoff B" für beliebige Wirkstoffe. Insbesondere ist Wirkstoff A ein Insulin, und Wirkstoff B ist ein GLP-1-Agonist. Wirkstoff A kann auch ein GLP-1-Agonist sein, und Wirkstoff B kann auch ein Insulin sein.

1. Vergleichsbeispiel

Bereitgestellt werden für eine Kombinationstherapie mit einem Wirkstoff A (z.B. ein Insulin) und einem Wirkstoff B (z.B. ein GLP-1-Agonist) ein Behälter 1 mit einer Zusammensetzung mit Wirkstoff A in einer Konzentration von a mg/mL und ein Behälter 2 mit einer Zusammensetzung mit Wirkstoff B in einer Konzentration von b mg/mL.

Zur Verabreichung einer Kombination der beiden Wirkstoffe wird ein Volumen $V_1$ mL aus Behälter 1 und ein Volumen $V_2$ mL aus Behälter 2 gemischt.

Für die Dosierung der beiden Wirkstoffe werden bei gegebenen Konzentrationen a und b die zu verabreichenden Volumina $V_1$ und $V_2$ abhängig von der zu verabreichenden Menge der Wirkstoffe A und B gewählt. Die Volumina $V_1$ und $V_2$ der beiden Wirkstoffe bestimmen sich anhand der Wirkstoffmengen wie folgt:

$$\text{Menge Wirkstoff A:} \quad V_1 \cdot a \text{ mg}$$
$$\text{Menge Wirkstoff B:} \quad V_2 \cdot b \text{ mg}$$

Die Konzentrationen der Wirkstoffe A und B in der Mischung der beiden Zusammensetzungen bestimmen sich wie folgt.

$$\text{Wirkstoff A:} \quad x \text{ mg/mL} = V_1 \cdot a / (V_1 + V_2)$$
$$\text{Wirkstoff B:} \quad y \text{ mg/mL} = V_2 \cdot b / (V_1 + V_2)$$

$V_1 + V_2$ ist das gesamte verabreichte Volumen. Dies bedeutet, dass sich die beiden Wirkstoffe gegenseitig verdünnen. Damit ist es mit dieser Anordnung nicht möglich, z.B. die Konzentration des Wirkstoffs A (z.B. des Insulins) bei wechselnden Mengen von Wirkstoff B auf einem vorbestimmten Wert zu halten.

2. Erfindungsgemäßes Beispiel

In diesem Beispiel werden für eine Kombinationstherapie mit einem Wirkstoff A (z.B. ein Insulin) und einem Wirkstoff B (z.B. ein GLP-1-Agonist) ein Behälter 1 mit einer Zusammensetzung mit Wirkstoff A in einer Konzentration von a mg/mL und ein Behälter 2 mit einer Zusammensetzung mit Wirkstoff A in einer Konzentration von a mg/ml und mit Wirkstoff B in einer Konzentration von b mg/mL bereitgestellt. Die Konzentration des Wirkstoffs A ist also in beiden Zusammensetzungen gleich.

Zur Verabreichung einer Kombination der beiden Wirkstoffe wird ein Volumen $V_3$ mL aus Behälter 1 und ein Volumen $V_2$ mL aus Behälter 2 gemischt.

Für die Dosierung der beiden Wirkstoffe werden bei gegebenen Konzentrationen a und b die zu verabreichenden Volumina $V_3$ und $V_2$ abhängig von der zu verabreichenden Menge der Wirkstoffe A und B gewählt. Die Volumina $V_3$ und $V_2$ der beiden Wirkstoffe bestimmen sich anhand der Wirkstoffmengen wie folgt:

$$\text{Menge Wirkstoff A: } (V_3 \cdot a + V_2 \cdot a) \text{ mg}$$

$$\text{Menge Wirkstoff B: } V_2 \cdot b \text{ mg}$$

Die Konzentrationen der Wirkstoffe A und B bestimmen sich wie folgt.

$$\text{Wirkstoff A:} \quad a \, mg/mL = (V_3 \cdot a + V_2 \cdot a)/(V_3 + V_2)$$
$$\text{Wirkstoff B:} \quad z \, mg/mL = V_2 \cdot b / (V_3 + V_2)$$

$V_3 + V_2$ ist das gesamte verabreichte Volumen. Aus der obigen Berechnung ergibt sich, dass die Konzentration des Wirkstoffs A immer a mg/ml beträgt, also konstant ist, egal, welches Volumenverhältnis $V_3/V_2$ dosiert wird.

Vergleicht man das Vergleichsbeispiel (siehe Punkt 1) mit dem vorliegenden erfindungsgemäßen Beispiel, so ergibt sich, dass bei gleicher Dosierungsmenge der Wirkstoffe A und B im erfindungsgemäßen Beispiel ein geringeres Gesamtvolumen benötigt wird.

Für eine gegebene Dosis (Wirkstoffmenge) des Wirkstoffs A gilt im

Vergleichsbeispiel: $V_1 \cdot a$ mg

Im erfindungsgemäßen Beispiel gilt: $(V_3 \cdot a + V_2 \cdot a)$ mg

Da in beiden Fällen die Wirkstoffmenge gleich sein soll, gilt

$$(V_3 \cdot a + V_2 \cdot a) = V_1 \cdot a$$

$$(V_3 + V_2) \cdot a = V_1 \cdot a$$

und

$$V_3 + V_2 = V_1$$

oder

$$V_3 = V_1 - V_2$$

Hierbei ist das Volumen $V_2$, in dem der Wirkstoff B gegeben wird, in beiden Fällen gleich.

Das Gesamtvolumen im Vergleichsbeispiel beträgt $V_1 + V_2$

Das Gesamtvolumen im erfindungsgemäßen Beispiel beträgt $V_3 + V_2$

Nach der obigen Gleichung gilt für das erfindungsgemäße Beispiel:

$$V_3 + V_2 = V_1 - V_2 + V_2 = V_1$$

Dieses Volumen $V_1$ ist kleiner als das Volumen $V_1 + V_2$ des Vergleichsbeispiels.

Durch die Mischung der Zusammensetzung mit Wirkstoff A und B mit der Zusammensetzung mit Wirkstoff A wird der Wirkstoff B verdünnt. Diese Verdünnung ist geringer als die Verdünnung des Wirkstoffs B im Vergleichsbeispiel (d. h. die Konzentration b > Konzentration z > Konzentration y):

$$b \quad > z$$

$$b \quad > V_2 \cdot b / (V_3 + V_2)$$

$$b \quad > b \cdot V_2 / (V_3 + V_2), \text{ wobei } V_2 / (V_3 + V_2) < 1 \text{ ist,}$$

und

$$z \qquad > y$$

$$V_2 \cdot b / (V_3 + V_2) \qquad > V_2 \cdot b / (V_1 + V_2)$$

$$1 / (V_3 + V_2) \qquad > 1 / (V_1 + V_2)$$

$$1 / (V_1 - V_2 + V_2) \qquad > 1 / (V_1 + V_2)$$

$$1 / V_1 \qquad > 1 / (V_1 + V_2)$$

Damit weist das erfindungsgemäße Dosierungssystem zur Verabreichung variabler Dosen der Wirkstoffe A (z.B. ein Insulin) und B (z.B. ein GLP-1-Agonist) drei Vorteile gegenüber dem Vergleichssystem auf:

- Die Konzentration des Wirkstoffs A (z. B. eines Insulins) kann konstant auf einem vorbestimmten Wert gehalten werden
- Bei gleichen zu verabreichenden Dosen der Wirkstoffe A und B ist das zu verabreichende Gesamtvolumen ist geringer.
- Die Verdünnung des Wirkstoffs B (z.B. des GLP-1-Agonisten) ist geringer als im Vergleichsversuch. Damit kann die Konzentration des Wirkstoffs B leichter in einem vorbestimmten Bereich gehalten werden.

Das vorliegende Beispiel kann ohne weiteres auf Arzneimittel mit drei oder mehr Wirkstoffen erweitert werden, wobei der erste Wirkstoff in allen Zusammensetzungen enthalten ist (vorzugsweise in gleichen Gewichtsanteilen) und in jeder weiteren Zusammensetzung mindestens ein weiterer Wirkstoff enthalten ist. Die erste Zusammensetzung kann mit jeder weiteren Zusammensetzung in jedem Verhältnis gemischt werden, ohne dass die Konzentration des Wirkstoffs in der ersten Zusammensetzung verdünnt wird.

## Patentansprüche

1. Pharmazeutische Kombination umfassend (a) Gly(A21)-Arg(B31)-Arg(B32)-Humaninsulin oder/und ein pharmakologisch tolerierbares Salz davon in einer Konzentration von 40 bis 500 U/ml, und (b) desPro$^{36}$Exendin-4(1-39)-Lys$_6$-NH$_2$ oder/und ein pharmakologisch tolerierbares Salz davon in einer Konzentration von 10 bis 300 μg/ml.

2. Pharmazeutische Kombination nach Anspruch 1, umfassend Gly(A21)-Arg(B31)-Arg(B32)-Humaninsulin oder/und ein pharmakologisch tolerierbares Salz davon in einer Konzentration von 100 bis 500 U/ml.

3. Pharmazeutische Kombination nach Anspruch 1 oder 2, umfassend desPro$^{36}$Exendin-4(1-39)-Lys$_6$-NH$_2$ oder/und ein pharmakologisch tolerierbares Salz davon in einer Konzentration von 20 bis 150 μg/ml.

4. Pharmazeutische Kombination nach einem der Ansprüche 1 bis 3, zur Verwendung zur Behandlung von Typ 2 Diabetes mellitus.

5. Pharmazeutische Kombination nach einem der Ansprüche 1 bis 3 oder zur Verwendung nach Anspruch 4 zur Verwendung zur Einstellung der nüchternen, der postprandialen oder/und der postabsorptiven Blutglukosekonzentration, zur Verbesserung der Glukosetoleranz, zur Prävention einer Hypoglykämie, zur Prävention eines Funktionsverlustes der β-Zellen des Pankreas, zur Gewichtsabnahme oder/und zur Prävention einer Gewichtszunahme.

6. Pharmazeutische Zusammensetzung umfassend (a) Gly(A21)-Arg(B31)-Arg(B32)-Humaninsulin oder/und ein pharmakologisch tolerierbares Salz davon in einer Konzentration von 40 bis 500 U/ml, und (b) desPro$^{36}$Exendin-4(1-39)-Lys$_6$-NH$_2$ oder/und ein pharmakologisch tolerierbares Salz davon in einer Konzentration von 10 bis 300 µg/ml.

**7.** Pharmazeutische Zusammensetzung nach Anspruch 6, umfassend Gly(A21)-Arg(B31)-Arg(B32)-Humaninsulin oder/und ein pharmakologisch tolerierbares Salz davon in einer Konzentration von 100 bis 500 U/ml.

**8.** Pharmazeutische Zusammensetzung nach Anspruch 6 oder 7, umfassend desPro$^{36}$Exendin-4(1-39)-Lys$_6$-NH$_2$ oder/und ein pharmakologisch tolerierbares Salz davon in einer Konzentration von 20 bis 150 µg/ml.

**9.** Pharmazeutische Zusammensetzung nach einem der Ansprüche 6 bis 8, zur Verwendung zur Behandlung von Typ 2 Diabetes mellitus.

**10.** Pharmazeutische Zusammensetzung nach einem der Ansprüche 6 bis 8 oder zur Verwendung nach Anspruch 9, zur Verwendung zur Einstellung der nüchternen, der postprandialen oder/und der postabsorptiven Blutglukosekonzentration, zur Verbesserung der Glukosetoleranz, zur Prävention einer Hypoglykämie, zur Prävention eines Funktionsverlustes der β-Zellen des Pankreas, zur Gewichtsabnahme oder/und zur Prävention einer Gewichtszunahme.

**11.** Verfahren zur Herstellung einer Zusammensetzung gemäß einem der Ansprüche 6 bis 10, umfassend Formulieren oder/und Konfektionieren von Gly(A21)-Arg(B31)-Arg(B32)-Humaninsulin und desPro$^{36}$Exendin-4(1-39)-Lys$_6$-NH$_2$.

**12.** Vorrichtung, umfassend die Kombination gemäß einem der Ansprüche 1 bis 5 oder die Zusammensetzung gemäß einem der Ansprüche 6 bis 10.

**13.** Vorrichtung gemäß Anspruch 12, zur Injektion.

**14.** Kit, umfassend die Kombination gemäß einem der Ansprüche 1 bis 5 oder die Zusammensetzung gemäß einem der Ansprüche 6 bis 10.

**Claims**

**1.** Pharmaceutical combination comprising (a) Gly(A21)-Arg(B31)-Arg(B32)-human insulin or/and a pharmacologically tolerable salt thereof in a concentration of 40 to 500 U/ml, and (b) desPro$^{36}$exendin-4(1-39)-Lys$_6$-NH$_2$ or/and a pharmacologically tolerable salt thereof in a concentration of 10 to 300 µg/ml.

**2.** Pharmaceutical combination according to Claim 1, comprising Gly(A21)-Arg(B31)-Arg(B32)-human insulin or/and a pharmacologically tolerable salt thereof in a concentration of 100 to 500 U/ml.

**3.** Pharmaceutical combination according to Claim 1 or 2, comprising desPro$^{36}$exendin-4(1-39)-Lys$_6$-NH$_2$ or/and a pharmacologically tolerable salt thereof in a concentration of 20 to 150 µg/ml.

**4.** Pharmaceutical combination according to any of Claims 1 to 3 for use for treatment of type 2 diabetes mellitus.

**5.** Pharmaceutical combination according to any of Claims 1 to 3 or for use according to Claim 4 for use for adjusting the fasting, post-prandial or/and post-operative blood glucose concentration, for improving glucose tolerance, for preventing hypoglycaemia, for preventing a loss of function of the β cells of the pancreas, for weight loss or/and for preventing weight gain.

**6.** Pharmaceutical composition comprising (a) Gly(A21)-Arg(B31)-Arg(B32)-human insulin or/and a pharmacologically tolerable salt thereof in a concentration of 40 to 500 U/ml, and (b) desPro$^{36}$exendin-4(1-39)-Lys$_6$-NH$_2$ or/and a pharmacologically tolerable salt thereof in a concentration of 10 to 300 µg/ml.

**7.** Pharmaceutical composition according to Claim 6, comprising Gly(A21)-Arg(B31)-Arg(B32)-human insulin or/and a pharmacologically tolerable salt thereof in a concentration of 100 to 500 U/ml.

**8.** Pharmaceutical composition according to Claim 6 or 7, comprising desPro$^{36}$exendin-4(1-39)-Lys$_6$-NH$_2$ or/and a pharmacologically tolerable salt thereof in a concentration of 20 to 150 µg/ml.

**9.** Pharmaceutical composition according to any of Claims 6 to 8 for use for treatment of type 2 diabetes mellitus.

**10.** Pharmaceutical composition according to any of Claims 6 to 8 or for use according to Claim 9 for use for adjusting the fasting, post-prandial or/and post-operative blood glucose concentration, for improving glucose tolerance, for preventing hypoglycaemia, for preventing a loss of function of the β cells of the pancreas, for weight loss or/and for preventing weight gain.

**11.** Method of producing a composition according to any of Claims 6 to 10, comprising formulating or/and compounding Gly(A21)-Arg(B31)-Arg(B32)-human insulin and desPro$^{36}$exendin-4(1-39)-Lys$_6$-NH$_2$.

**12.** Device comprising the combination according to any of Claims 1 to 5 or the composition according to any of Claims 6 to 10.

**13.** Device according to Claim 12 for injection.

**14.** Kit comprising the combination according to any of Claims 1 to 5 or the composition according to any of Claims 6 to 10.

**Revendications**

**1.** Combinaison pharmaceutique comprenant (a) Gly(A21)-Arg(B31)-Arg(B32)-insuline humaine ou/et un sel pharmacologiquement tolérable correspondant en une concentration de 40 à 500 U/ml, et (b) desPro$^{36}$Exendine-4(1-39)-Lys$_6$-NH$_2$ ou/et un sel pharmacologiquement tolérable correspondant en une concentration de 10 à 300 μg/ml.

**2.** Combinaison pharmaceutique selon la revendication 1, comprenant Gly(A21)-Arg(B31)-Arg(B32)-insuline humaine ou/et un sel pharmacologiquement tolérable correspondant en une concentration de 100 à 500 U/ml.

**3.** Combinaison pharmaceutique selon la revendication 1 ou 2, comprenant desPro$^{36}$Exendine-4(1-39)-Lys$_6$-NH$_2$ ou/et un sel pharmacologiquement tolérable correspondant en une concentration de 20 à 150 μg/ml.

**4.** Combinaison pharmaceutique selon l'une quelconque des revendications 1 à 3, pour une utilisation pour le traitement du diabète sucré de type 2.

**5.** Combinaison pharmaceutique selon l'une quelconque des revendications 1 à 3 ou pour l'utilisation selon la revendication 4, pour une utilisation pour l'ajustement de la concentration de glucose dans le sang à jeun, postpandriale ou/et post-absorptive, pour l'amélioration de la tolérance au glucose, pour la prévention d'une hypoglycémie, pour la prévention d'une perte de fonction des cellules β du pancréas, pour la perte de poids ou/et pour la prévention d'une prise de poids.

**6.** Composition pharmaceutique comprenant (a) Gly(A21)-Arg(B31)-Arg(B32)-insuline humaine ou/et un sel pharmacologiquement tolérable correspondant en une concentration de 40 à 500 U/ml, et (b) desPro$^{36}$Exendine-4(1-39)-Lys$_6$-NH$_2$ ou/et un sel pharmacologiquement tolérable correspondant en une concentration de 10 à 300 μg/ml.

**7.** Composition pharmaceutique selon la revendication 6, comprenant Gly(A21)-Arg(B31)-Arg(B32)-insuline humaine ou/et un sel pharmacologiquement tolérable correspondant en une concentration de 100 à 500 U/ml.

**8.** Composition pharmaceutique selon la revendication 6 ou 7, comprenant desPro$^{36}$Exendine-4(1-39)-Lys$_6$-NH$_2$ ou/et un sel pharmacologiquement tolérable correspondant en une concentration de 20 à 150 μg/ml.

**9.** Composition pharmaceutique selon l'une quelconque des revendications 6 à 8 pour une utilisation pour le traitement du diabète sucré de type 2.

**10.** Composition pharmaceutique selon l'une quelconque des revendications 6 à 8 ou pour l'utilisation selon la revendication 9, pour une utilisation pour l'ajustement de la concentration de glucose dans le sang à jeun, postpandriale ou/et post-absorptive, pour l'amélioration de la tolérance au glucose, pour la prévention d'une hypoglycémie, pour la prévention d'une perte de fonction des cellules β du pancréas, pour la perte de poids ou/et pour la prévention d'une prise de poids.

**11.** Procédé pour la préparation d'une composition selon l'une quelconque des revendications 6 à 10, comprenant la formulation ou/et la confection de Gly(A21)-Arg(B31)-Arg(B32)-insuline humaine et de desPro$^{36}$Exendine-4(1-39)-Lys$_6$-NH$_2$.

**12.** Dispositif, comprenant la combinaison selon l'une quelconque des revendications 1 à 5 ou la composition selon l'une quelconque des revendications 6 à 10.

**13.** Dispositif selon la revendication 12, pour une injection.

**14.** Kit, comprenant la combinaison selon l'une quelconque des revendications 1 à 5 ou la composition selon l'une quelconque des revendications 6 à 10.

Fig. 1

EP 3 228 320 B1

Fig. 2

Fig. 3

Fig. 4

EP 3 228 320 B1

Fig. 5a

Fig. 5b

Fig. 6

Fig. 7

M080212db

Fig. 8

Fig. 9

| Lantus-Dosis | Mix A | AVE10-Dosis Mix B | Mix C |
|---|---|---|---|
| 10 | 6.7 | | |
| 12 | 8.0 | | |
| 14 | 9.3 | | |
| 16 | 10.7 | | |
| 18 | 12.0 | | |
| 20 | 13.3 | | |
| 22 | 14.7 | 8.8 | |
| 24 | 16.0 | 9.6 | |
| 26 | 17.3 | 10.4 | |
| 28 | 18.7 | 11.2 | |
| 30 | 20.0 | 12.0 | |
| 32 | | 12.8 | |
| 34 | | 13.6 | |
| 36 | | 14.4 | |
| 38 | | 15.2 | |
| 40 | | 16.0 | |
| 42 | | 16.8 | 10.5 |
| 44 | | 17.6 | 11.0 |
| 46 | | 18.4 | 11.5 |
| 48 | | 19.2 | 12.0 |
| 50 | | 20.0 | 12.5 |
| 52 | | | 13.0 |
| 54 | | | 13.5 |
| 56 | | | 14.0 |
| 58 | | | 14.5 |
| 60 | | | 15.0 |
| 62 | | | 15.5 |
| 64 | | | 16.0 |
| 66 | | | 16.5 |
| 68 | | | 17.0 |
| 70 | | | 17.5 |
| 72 | | | 18.0 |
| 74 | | | 18.5 |
| 76 | | | 19.0 |
| 78 | | | 19.5 |
| 80 | | | 20.0 |

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0214826 A
- EP 0375437 A
- EP 0678522 A
- EP 0885961 A
- WO 2005046716 A
- EP 0419504 A
- WO 9200321 A
- EP 0368187 A
- DE 102008003568
- DE 102008003566
- WO 2009087081 A
- WO 2006051103 A
- WO 2003020201 A
- US 5424286 A
- WO 9805351 A
- WO 9907404 A
- WO 9925727 A
- WO 9925728 A
- WO 2004005342 A
- US 4689042 A
- US 5478323 A
- US 5253785 A
- WO 0102039 A
- WO 9808871 A
- WO 0104156 A
- WO 9830231 A
- EP 99610043 A
- WO 04035623 A

**In der Beschreibung aufgeführte Nicht-Patentliteratur**

- The Diabetes Control and Complications Trial Research Group. *N. Engl. J. Med.,* 1993, vol. 329, 977-986
- **HOLST.** *Curr. Med. Chem,* 1999, vol. 6, 1005
- **NAUCK et al.** *Exp Clin Endocrinol Diabetes,* 1997, vol. 105, 187
- **LOPEZ-DELGADO et al.** *Endocrinology,* 1998, vol. 139, 2811
- **HEINRICH et al.** *Endocrinol.,* 1984, vol. 115, 2176
- **UTTENTHAL et al.** *J Clin Endocrinol Metabol,* 1985, vol. 61, 472
- **GOKE et al.** *J. Biol Chem,* vol. 268, 19650-55
- **RAUFMAN.** *Reg. Peptides,* 1996, vol. 61, 1-18
- **TEWS et al.** *Horm Metab Res,* 2008, vol. 40, 172-180